# EUROPÄISCHE PATENTSCHRIFT

(11) **EP 0 871 362 B1**
(45) Veröffentlichungstag und Bekanntmachung des Hinweises auf die Patenterteilung: **17.04.2002**
(21) Anmeldenummer: 96943971.0
(22) Anmeldetag: 16.12.1996
(51) Int. Cl.: A01N 51/00, A01N 47/04, A01N 47/02

(54) **SYNERGISTISCHE INSEKTIZIDE MISCHUNGEN**
SYNERGISTIC INSECTICIDE MIXTURES
MELANGES INSECTICIDES SYNERGIQUES

(30) Priorität: 27.12.1995 DE 19548872
(43) Veröffentlichungstag der Anmeldung: 21.10.1998
(73) Patentinhaber: BAYER AG, 51368 Leverkusen (DE)
(72) Erfinder: UHR, Hermann, D-47800 Krefeld (DE); BUSCHHAUS, Hans-Ulrich, D-47800 Krefeld (DE); KUGLER, Martin, D-42799 Leichlingen (DE); KUNISCH, Franz, D-51519 Odenthal (DE); SCHRAGE, Heinrich, D-47829 Krefeld (DE)
(86) Internationale Anmeldenummer: EP9605644
(87) Internationale Veröffentlichungsnummer: WO9724032

(56) Entgegenhaltungen:
- WO-A-95/22902
- WO-A-96/23411
- WO-A-96/37105
- DATABASE WPI Section Ch, Week 8827 Derwent Publications Ltd., London, GB; Class C02, AN 88-188016 XP002030126 & JP 63 126 806 A (NIHON TOKUSHU NOYAKU SEIZO KK) , 30.Mai 1988

## Beschreibung

Die Erfindung betrifft synergistische insektizide Mischungen aus Fipronil und Agonisten bzw. Antagonisten von nicotinergen Acetylcholinrezeptoren zum Schutz von technischen Materialien und als Pflanzenschutzmittel.

Es ist bereits bekannt geworden, daß man Fipronil zur Bekämpfung von Holzschädlingen verwenden kann (EP-295 117; US-PS 5 232 940). Ein Vorteil besteht in dem sehr geringen Dampfdruck dieser Verbindungen.

Für Fipronil allein werden jedoch relativ hohe Konzentrationen benötigt. Es gelingt nur sehr schwer, die für eine Wirkung nötigen Konzentrationen ausreichend tief ins zu schützende Holz zu transportieren.

Desweiteren ist auch bekannt geworden, daß man Agonisten und Antagonisten von nicotinergen Acetylcholinrezeptoren zur Bekämpfung von Insekten und Holzschädlingen verwenden kann. Um auch hier in tieferen Holzschichten noch eine Akutwirkung zu erreichen muß der Wirkstoff in relativ großen Konzentrationen eingesetzt werden. Subletale Dosen führen zwar in vielen Fällen zu einer Verhaltensänderung der Spezies, sind aber in vielen Fällen auch reversibel.

Weiterhin sind in der WO 96/23411 insektizide Mischungen beschrieben, die außer Fipronil eine der Chlornicotinyl-Verbindungen Acetamiprid, Nitenpyram oder Imidchloprid enthalten.

In der WO 96/37105 sind insektizide Mischungen von Chlornicotinyl-Insektiziden mit zahlreichen Synergisten, darunter Fipronil beschrieben. Eine Verwendung als Mittel zum Schutz von technischen Materialien wird nicht offenbart.

WO 96/23411 und WO 96/37105 gehören gemäß Art 54(3) EPÜ zum Stand der Technik.

Es wurde nun gefunden, daß Mischungen aus Fipronil und mindestens einem Agonisten bzw. Antagonisten von Acetylcholinrezeptoren der Formel (I) synergistisch wirksam sind und zum Schutz von technischen Materialien, insbesondere Holz vor Insektenbefall geeignet sind. Diese Mischungen eignen sich auch im Pflanzenschutz der Bekämpfung tierischer Schädlinge. Aufgrund dieses Synergismus können deutlich geringere Wirkstoffmengen verwendet werden, d.h. die Wirkung der Mischung ist größer als die Wirkung der Einzelkomponenten.

Bei den Agonisten und Antagonisten der nicotinergen Acetylcholinrezeptoren handelt es sich um bekannte Verbindungen, die bekannt sind aus folgenden Publikationen:
Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 686, 303 570, 302 833, 306 696, 189 972, 455 000, 135 956, 471 372, 302 389;
Deutsche Offenlegungsschriften Nr. 3 639 877, 3 712 307;
Japanische Offenlegungsschriften Nr. 03 220 176, 02 207 083, 63 307 857, 63 287 764, 03 246 283, 04 9371, 03 279 359, 03 255 072;
US-Patentschriften Nr. 5 034 524, 4 948 798,4 918 086, 5 039 686, 5 034 404;
PCT-Anmeldungen Nr. WO 91/17 659, 91/4965;
Französische Anmeldung Nr. 2 611 114;
Brasilianische Anmeldung Nr. 88 03 621.

Auf die in diesen Publikationen beschriebenen generischen Formeln und Definitionen sowie auf die darin beschriebenen einzelnen Verbindungen wird hiermit ausdrücklich Bezug genommen.

Diese Verbindungen werden zum Teil unter dem Begriff Nitromethylene und damit verwandte Verbindungen zusammengefaßt.

Diese Verbindungen lassen sich unter der allgemeinen Formel (I) zusammenfassen 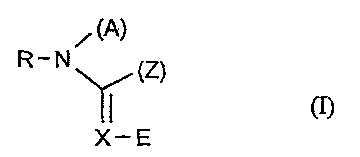 in welcher
- R: für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind, Hydroxy, Halogen, Cyano, Nitro, Amino, Monoalkyl- und Dialkylamino mit 1 bis 4 Kohlenstoffatomen je Alkylgruppe substituiertes Heteroarylmethyl mit bis zu 6 Ringatomen und N, O, S als Heteroatomen oder für Wasserstoff steht,
- A: für Wasserstoff oder Alkyl mit 1 bis 4 Kohlenstoffatomen steht,
- E: für NO₂ oder CN steht,
- X: für =CH- oder =N- steht,
- Z: für Alkyl oder für 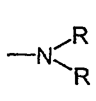 steht, und
- A und Z: gemeinsam mit den Atomen, an welche sie gebunden sind, einen gesättigten oder ungesättigten, 5- bis 7-gliedrigen heterocyclischen Ring bilden, der weitere 1 oder 2 gleiche oder verschiedene Heteroatome und/oder Heterogruppen enthalten kann, wobei als Heteroatome Sauerstoff, Schwefel oder Stickstoff und als Heterogruppen N-Alkyl in Frage kommen, wobei Alkyl der N-Alkylgruppe vorzugsweise 1 bis 4 Kohlenstoffatome enthält, mit Ausnahme der Verbindungen (E)-N¹-((6-chlor-3-pyridyl)methyl)-N²-cyano-N¹-methyl-acetamidine (Acetamiprid), (E)-N-((6-chlor-3-pyridyl)methyl)-N-ethyl-N'-methyl-2-nitrovinylidendiamin (Nitenpyram) und 1-(6-Chlor-3-pyridylmethyl)-N-nitroimidazolidin-2-ylidenamin (Imidachloprid).

Bevorzugt handelt es sich bei den Agonisten und Antagonisten der nicotinergen Acetylcholinrezeptoren um folgende Verbindungen:

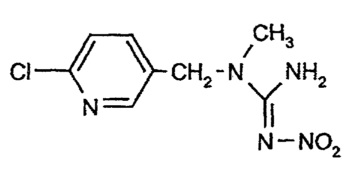

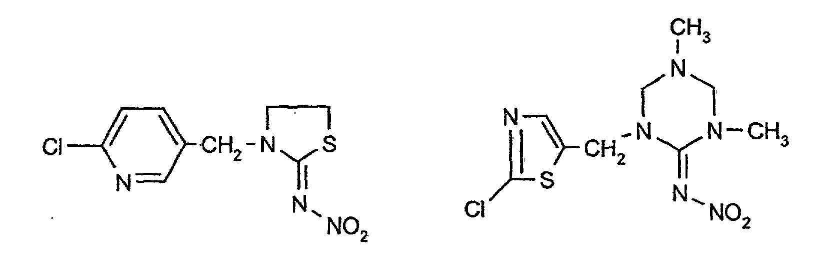

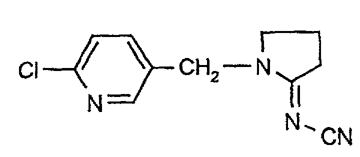

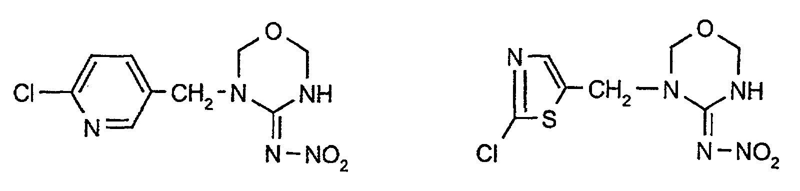

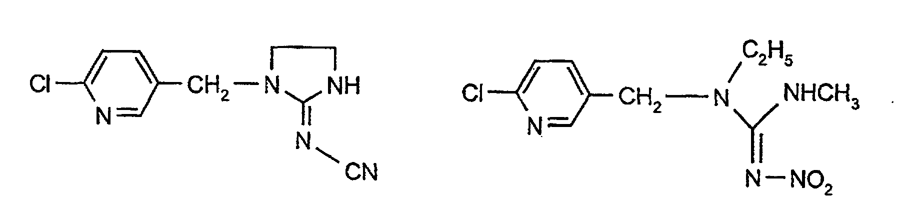

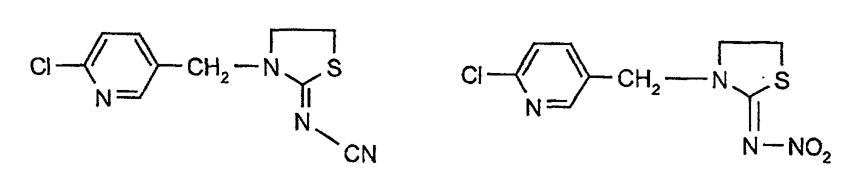

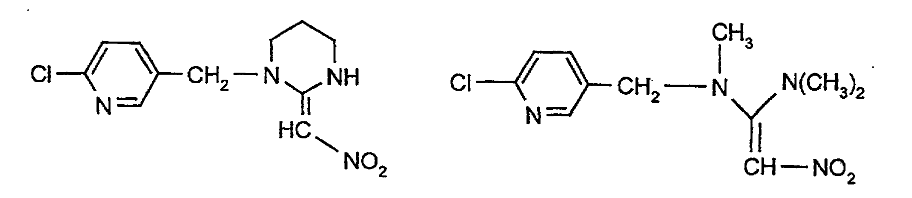

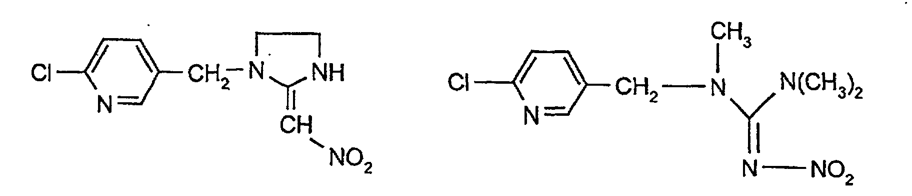

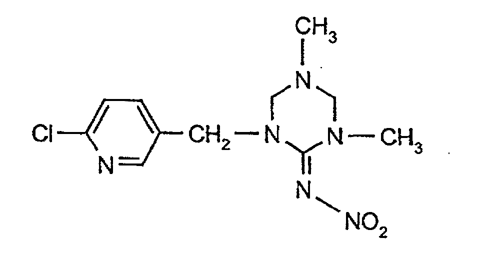

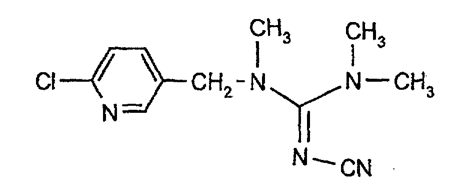

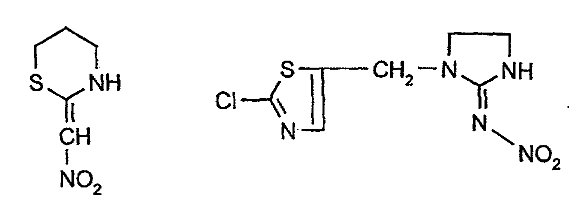

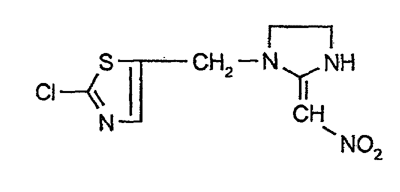

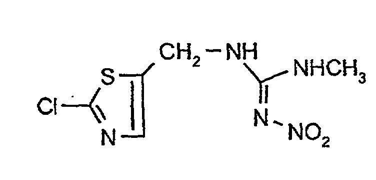

Ganz besonders bevorzugte Agonisten und Antagonisten der nicotinergen Acetylcholinrezeptoren sind Verbindungen der folgenden Formeln:

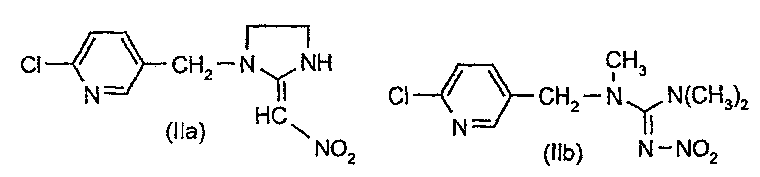

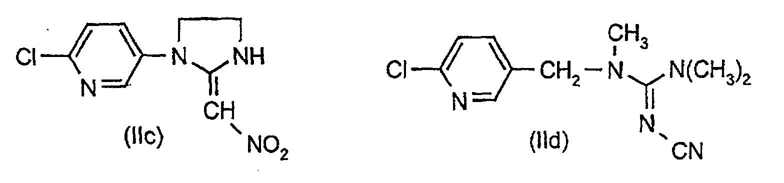

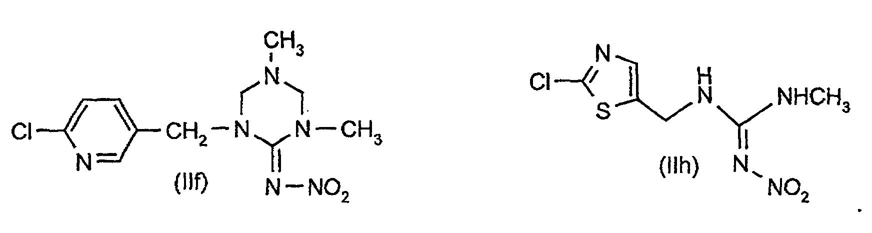

Die Wirkstoffkombination aus Fipronil und Agonisten und Antagonisten von nicotinergen Acetylcholinrezeptoren der Formel (I) eignen sich hervorragend zum Schutz von technischen Materialien, insbesondere von Holz vor dem Befall durch holzzerstörende Insekten, wie beispielsweise

### 1. Käfer

Hylotrupes bajulus, Chlorophorus pilosis, Anabium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.

### 2. Hautflügler

Sirex juvencus, Urocerus gigas, Urocerus gigas taignus, Urocerus augur.

### 3. Termiten

Kalotennes flavicollis, Cryptotermes brevis, Heterotermes indicola, Reticulitermes flavipes, Reticulitermes santonensis, Reticulitermes lucilugus, Mastotermes darwiniensis, Zootermopsis nevadensis, Coptotermes formosanus.

Das Verhältnis der eingesetzten Verbindungen der Formeln (I) und Fipronil, sowie die Gesamtmenge der Mischung ist von der Art und dem Vorkommen der Insekten abhängig. Die optimalen Verhältnisse und Gesamteinsatzmengen können bei jeder Anwendung jeweils durch Testreihen ermittelt werden. Im allgemeinen ist das Verhältnis der Verbindungen der allgemeinen Formeln (I) und Fipronil 1:100 bis 100:1, vorzugsweise 1:10 bis 10:1.

Die erfindungsgemäßen Wirkstoffkombinationen können generell in alle Holzschutzmittel bzw. -formulierungen eingearbeitet werden z.B. durch Vermischen der Wirkstoffe mit Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln oder als Zusatz zu beliebigen anderen Holzschutzformulierungen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und mindestens einen Emulgator und/oder Netzmittel oder besteht daraus.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet

Die organischen schwerflüchtigen öligen oder ölartigen Lösungsmitte) mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw, modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw, modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.%, vorzugsweise 50 bis 68 Gew. %, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon, Aminen wie z.B. Alkanolamine wie Monoethanolamin oder Ammoniak.

Unter Holz, welches durch die erfindungsgemäße Wirkstoffmischung bzw. diese enthaltende Mittel geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Ein besonders effektiver Holzschutz wird durch großtechnische Imprägnierverfahren, z.B. Vakuum, Doppelvakuum oder Druckverfahren, erzielt.

Die zum Schutz von Holz und Holzwerkstoffen verwendeten insektiziden Mittel oder Konzentrate enthalten die Wirkstoffkombination in einer Konzentration von 0,001 bis 95 Gew.-%, insbesondere 0,001 bis 60 Gew.-%.

Die erfindungsgemäßen Mittel ermöglichen in vorteilhafter Weise, die bisher verfügbaren insektiziden Mittel durch effektivere zu ersetzen. Sie zeigen eine gute Stabilität und haben in vorteilhafter Weise ein breites insektizides Wirkungsspektrum.

In gebrauchsfertigen Anwendungen können die erfindungsgemäßen Mischungen gegebenenfalls auch mit weiteren Insektiziden und zum zusätzlichen Erreichen einer Wirkung gegen holzzerstörende und holzverfärbende Pilze gegebenenfalls auch mit einem oder mehreren Fungiziden gemischt sein. In vielen Fällen sind dann noch zusätzliche Synergismen zu beobachten.

Als Insektizide, die gegebenenfalls zugemischt werden können handelt es sich beispielsweise um:
Phosphorsäureester wie Azinphos-ethyl, Azinphos-methyl, α-1(4-Chlorphenyl)-4-(O-ethyl, S-propyl)phosphoryloxy-pyrazol, Chlorpyrifos, Coumaphos, Demeton, Demeton-S-methyl, Diazinon, Dichlorvos, Dimethoate, Ethoate, Ethoprophos, Etrimfos, Fenitrothion, Fenthion, Heptenophas, Parathion, Parathion-methyl, Phosalone, Phoxim, Pirimiphos-ethyl, Pirimiphos-methyl, Profenofos, Prothiofos, Sulfprofos, Triazophos und Trichlorphon;
Carbamate wie Aldicarb, Bendiocarb, α-2-(1-Methylpropyl)-phenylmethylcarbamat, Butocarboxim, Butoxycarboxim, Carbaryl, Carbofuran, Carbosulfan, Cloethocarb, Isoprocarb, Methomyl, Oxamyl, Pirimicarb, Promecarb, Propoxur und Thiodicarb;
Organosiliciumverbindungen, vorzugsweise Dimethyl(phenyl)silyl-methyl-3-phenoxybenzylether wie Dimethyl-(4-ethoxyphenyl)-silylmethyl-3-phenoxybenzylether oder (Dimethylphenyl)-silyl-methyl-2-phenoxy-6-pyridylmethylether wie z.B. Dimethyl-(9-ethoxy-phenyl)-silylmethyl-2-phenoxy-6-pyridylmethylether oder [(Phenyl)-3-(3-phenoxyphenyl)-propyl](dimethyl)-silane wie z.B. (4-Ethoxyphenyl)-[3-(4-fluoro-3-phenoxyphenyl-propyl]dimethyl-silan, Silafluofen;
Pyrethroide wie Allethrin, Alphamethrin, Bioresmethrin, Byfenthrin, Cycloprothrin, Cyfluthrin, Decamethrin, Cyhalothrin, Cypermethrin, Deltamethrin, Alpha-cyano-3-phenyl-2-methylbenzyl-2,2-dimethyl-3-(2-chlor-2-trifluor-methylvinyl)cyclopropancarboxylat, Fenpropathrin, Fenfluthrin, Fenvalerate, Flucythrinate, Flumethrin, Fluvalinate, Permethrin, Resmethrin und Tralomethrin;
Nitroimine und Nitromethylene wie 1-[(6-Chlor-3-pyridinyl)-methyl]-4,5-dihydro-N-nitro-1H-imidazol-2-amin (Imidacloprid), N-[(6-Chlor-3-pyridyl)methyl-]N²cyano-N¹-methylacetamide (NI-25);
Abamectin, AC 303.630, Acephate, Acrinathrin, Alanycarb, Aldoxycarb, Aldrin, Amitraz, Azamethiphos, Bacillus thuringiensis, Phosmet, Phosphamidon, Phosphine, Prallethrin, Propaphos, Propetamphos, Prothoate, Pyraclofos, Pyrethrins, Pyridaben, Pyridafenthion, Pyriproxyfen, Quinalphos, RH-7988, Rotenone, Sodium fluoride, Sodium hexafluorosilicate, Sulfotep, Sulfuryl fluoride, Tar Oils, Teflubenzuron, Tefluthrin, Temephos, Terbufos, Tetrachlorvinphos, Tetramethrin, O-2-tert.-Butyl-pyrimidin-5-yl-o-isopropyl-phosphorothiate, Thiocyclam, Thiofanox, Thiometon, Tralomethrin, Triflumuron, Trimethacarb, Vamidothion, Verticillium Lacanii, XMC, Xylylcarb, Benfuracarb, Bensultap, Bifenthrin, Bioallethrin, MERbioallethrin (S)-cyclopentenyl isomer, Bromophos, Bromophos-ethyl, Buprofezin, Cadusafos, Calcium Polysulfide, Carbophenothion, Cartap, Chinomethionat, Chlordane, Chlorfenvinphos, Chlorfluazuron, Chlormephos, Chloropicrin, Chlorpyrifos, Cyanophos, Beta-Cyfluthrin, Alpha-cypermethrin, Cyophenothrin, Cyromazine, Dazomet, DDT, Demeton-S-methylsulphon, Diafenthiuron, Dialifos, Dicrotophos, Diflubenzuron, Dinoseb, Deoxabenzofos, Diaxacarb, Disulfoton, DNOC, Empenthrin, Endosulfan, EPN, Esfenvalerate, Ethiofencarb, Ethion, Etofenprox, Fenobucarb, Fenoxycarb, Fensulfothion, Fipronil, Flucycloxuron, Flufenprox, Flufenoxuron, Fonofos, Formetanate, Formothion, Fosmethilan, Furathiocarb, Heptachlor, Hexaflumuron, Hydramethylnon, Hydrogen Cyanide, Hydroprene, IPSP, Isazofos, Isofenphos, Isoprothiolane, Isoxathion, lodfenphos, Kadethrin, Lindane, Malathion, Mecarbam, Mephosfolan, Mercurous, chloride, Metam, Metarthizium, anisopliae, Methacrifos, Methamidophos, Methidathion, Methiocarb, Methoprene, Methoxychlor, Methyl isothiocyanate, Metholcarb, Mevinphos, Monocrotophos, Naled, Neodiprion sertifer NPV, Nicotine, Omethoate, Oxydemeton-methyl, Pentachlorophenol, Petroleum oils, Phenothrin, Phenthoate, Phorate.

Dabei können die gegebenenfalls noch zumischbaren weiteren Insektizide auch aus der Klasse der Verbindungen der allgemeinen Formel (I) stammen.

Als gegebenenfalls noch zumischbaren Fungizide kommen vorzugsweise in Frage:

### Triazole wie:

Azaconazole, Propiconazole, Tebuconazole, Cyproconazole, Metconazole, Amitrole, Azocyclotin, BAS 480F, Bitertanol, Difenoconazole, Fenbuconazole, Fenchlorazole, Fenethanil, Fluquinconazole, Flusilazole, Flutriafol, Imibenconazole, Isozofos, Myclobutanil, Paclobutrazol, (±)-cis-1-(4-chlorphenyl)-2-(1H-1,2,4-triazol-1-yl)-cycloheptanol, Tetraconazole, Triadimefon, Triadimenol, Triapenthenol, Triflumizole, Triticonazole, Uniconazole sowie deren Metallsalze und Säureaddukte.

### Imidazole wie:

Imazalil, Pefurazoate, Prochloraz, Triflumizole, 2-(1-tert-Butyl)-1-(2-chlorphenyl)-3-(1,2,4-triazol-1-yl)-propan-2-ol, Thiazolcarboxanilide wie 2',6'-Dibromo-2-methyl-4-trifluoromethoxy-4'-trifluoromethyl-1,3-thiazole-5-carboxanilide, 1-Imidazolyl-1-(4'-chlorophenoxy)-3,3-dimethylbutan-2-on sowie deren Metallsalze und Säureaddukte.

Methyl(E)-2-[2-[6-(2-cyanophenoxy)pyrimidin-4-yloxy]phenyl]3-methoxyacrylate, methyl(E)-2-[2-[6-(2-thioamidophenoxy)pyrimidin-4-yloxy]phenyl]-3-methoxyacrylate, methyl(E)-2-[2-[6-(2-fluorophenoxy)pyrimidin-4-yloxy]phenyl]-3-methoxyacrylate, methyl(E)-2-[2-[6-(2,6-difluorophenoxy)pyrimidin-4-yloxy]phenyl]-3-methoxyacrylate, methyl(E)-2-[2-[3-(pyrimidin-2-yloxy)phenoxy]phenyl]-3-methoxyacrylate, methyl(E)-2-[2-[3-(5-methylpyrimidin-2-yloxy)-phenoxy]phenyl]-3-methoxyacrylate, methyl(E)-2-[2-[3-(phenyl-sulfonyloxy)phenoxy]phenyl]-3-methoxyacrylate, methyl(E)-2-[2-[3-(4-nitrophenoxy)phenoxy]phenyl]-3-methoxyacrylate, methyl(E)-2-[2-phenoxyphenyl]-3-methoxyacrylate, methyl(E)-2-[2-(3,5-dimethylbenzoyl)pyrrol-1-yl]-3-methoxyacrylate, methyl(E)-2-[2-(3-methoxyphenoxy)phenyl]-.3-methoxyacrylate, methyl(E)-2-[2-(2-phenylethen-1-yl)-phenyl]-3-methoxyacrylate, methyl(E)-2-[2-(3,5-dichlorophenoxy)pyridin-3-yl]-3-methoxyacrylate, methyl(E)-2-(2-(3-(1,1,2,2-tetrafluoroethoxy)phenoxy)phenyl)-3-methoxyacrylate, methyl(E)-2-(2-[3-(alpha-hydroxybenzyl)phenoxy]phenyl)-3-methoxyacrylate, methyl(E)-2-(2-(4-phenoxypyridin-2-yloxy)phenyl)-3-methoxyacrylate, methyl(E)-2-[2-(3-n-propyloxyphenoxy)phenyl]3-methoxyacrylate, methyl(E)-2-[2-(3-isopropyloxyphenoxy)phenyl]-3-methoxyacrylate, methyl(E)-2-[2-[3-(2-fluorophenoxy)pehnoxy]phenyl]-3-methoxyacrylate, methyl(E)-2-[2-(3-ethoxyphenoxy)-phenyl]-3-methoxyacrylate, methyl(E)-2-[2-(4-tert.-butylpyridin-2-yloxy)phenyl]-3-methoxyacrylate, methyl(E)-2-[2-[3-(3-cyanophenoxy)phenoxy]phenyl]-3-methoxyacrylate, methyl(E)-2-[2-(3-methylpyridin-2-yloxymethyl)phenyl]-3-methoxyacrylate, methyl(E)-2-[2-[6-(2-methylphenoxy)pyrimidin-4-yloxy]phenyl]-3-methoxyacrylate,methyl(E)-2-[2-(5-brom opyridin-2-yloxymethyl)phenyl]-3-methoxyacrylate, methyl(E)-2-[2-(3-(3-iodopyridin-2-yloxy)phenoxy)phenyl]-3-methoxyacrylate, methyl(E)-2-[2-[6-(2-chloropyridin-3-yloxy)pyrimidin-4-yloxy]phenyl]-3-methoxyacrylate, (E),(E)methyl-2-[2-(5,6-dimethylpyrazin-2-ylmethyloximinomethyl)phenyl]-3-methoxyacrylate, (E)-methyl-2-{2-[6-(6-methylpyridin-2-yloxy)pyrimidin-4-yloxy]phenyl}-3-methoxyacrylate, (E),(E)methyl-2-{2-(3-methoxyphenyl)-methyloximinomethyl]phenyl}-3-methoxyacrylate, (E)methyl-2-{2-(6-(2-azidophenoxy)-pyrimidin-4-yloxy]phenyl}3-methoxyacrylate, (E),(E)methyl-2-{2-[6-phenylpyrimidin-4-yl)-methyloximinomethyl]phenyl}-3-methoxyacrylate, (E),(E)methyl-2-{2-[(4-chlorophenyl)-methyloximinomethyl]phenyl}-3-methoxyacrylate, (E)methyl-2-{2-[6-(2-n-propylphenoxy)-1,3,5-triazin-4-yloxy]phenyl}-3-methoxyacrylate, (E),(E)methyl-2-{2-[(3-nitrophenyl)methyloximinomethyl]phenyl}-3-methoxyacrylate;

### Succinat-Dehydrogenase Inhibitoren wie:

Fenfuram, Furcarbanil, Cyclafluramid, Furmecyclox, Seedvax, Metsulfovax, Pyrocarbolid, Oxycarboxin, Shirlan, Mebenil (Mepronil), Benodanil, Flutolanil (Moncut);
Naphthalin-Derivate wie Terbinafine, Naftifine, Butenafine, 3-Chloro-7-(2-aza-2,7,7-trimethyl-oct-3-en-5-in);
Sulfenamide wie Dichlofluanid, Tolylfluanid, Folpet, Fluorfolpet; Captan, Captofol;
Benzimidazole wie Carbendazim, Benomyl, Furathiocarb, Fuberidazole, Thiophonatmethyl, Thiabendazole oder deren Salze;
Morpholinderivate wie Fenpropimorph, Falimorph, Dimethomorph, Dodemorph, Aldimorph, Fenpropidin und ihre arylsulfonsauren Salze, wie z.B. p-Toluolsulfonsäure und p-Dodecylphenyl-sulfonsäure;
Dithiocarbamate, Cufraneb, Ferbam, Mancopper, Mancozeb, Maneb, Metam, Metiram, Thiram Zeneb, Ziram;
Benzthiazole wie 2-Mercaptobenzothiazol;
Benzamide wie 2,6-Dichloro-N-(4-trifluoromethylbenzyl)-benzamide;
Borverbindungen wie Borsäure, Borsäureester, Borax;
Formaldehyd und Formaldehydabspaltende Verbindungen wie Benzylalkoholmono-(poly)-hemiformal, Oxazolidine, Hexa-hydro-S-triazine, N-Methylolchloracetamid, Paraformadehyd, Nitropyrin, Oxolinsäure, Tecloftalam;
Tris-N-(cyclohexyldiazeniumdioxy)-aluminium, N-(Cyclo-hexyldiazeniumdioxy)-tributylzinn bzw. K-Satze, Bis-N-(cyclohexyldiazeniumdioxy)-kupfer;
N-Methylisothiazolin-3-on, 5-Chlor-N-methylisothiazolin-3-on, 4,5-Dichloro-N-octylisothiazolin-3-on, N-Octyl-isothiazolin-3-on, 4,5-Trimethylen-isothiazolinone, 4,5-Benzisothiazolinone, N-Methylolchloracetamid;
Aldehyde wie Zimtaldehyd, Formaldehyd, Glutardialdehyd, β-Bromzimtaldehyd; Thiocyanate wie Thiocyanatomethylthiobenzothiazol, Methylenbisthiocyanat, usw; quartäre Ammoniumverbindungen wie Benzyldimethyltetradecylammoniumchlorid, Benzyldimethyldodecylammoniumchlorid, Didecyldimethaylammoniumchlorid;
Iodderivate wie Diiodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-butylcarbamat, 3-Iod-2-propinyl-n-hexylcarbamat, 3-Iod-2-propinyl-cyciohexylcarbamat, 3-Iod-2-propinyl-phenylcarbamat;
Phenolderivate wie Tribromphenol, Tetrachlorphenol, 3-Methyl-4-chlorphenol, 3,5-Dimethyl-4-chlorphenol, Phenoxyethanol, Dichlorphen, o-Phenylphenol, m-Phenylphenol, p-Phenylphenol, 2-Benzyl-4-chlorphenol und deren Alkali- und Erdalkalimetallsalze;
Mikrobizide mit aktivierter Halogengruppe wie Chloracetamid, Bronopol, Bronidox, Tectamer wie 2-Brom-2-nitro-1,3-propandiol, 2-Brom-4'-hydroxy-acetophenon, 2,2-Dibrom-3-nitril-propionamid, 1,2-Dibrom-2,4-dicyanobutan, β-Brom-β-nitrostyrol;
Pyridine wie 1-Hydroxy-2-pyridinthion (und ihre Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
Metallseifen wie Zinn-, Kupfer-, Zinknaphtenat, -octoat, 2-ethylhexanoat, -oleat, -phosphat, -benzoat;
Metallsalze wie Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat, insbesondere Mischung mit Fixiermitteln;
Oxide wie Tributylzinnoxid, Cu₂O, CuO, ZnO;
Dialkyldithiocarbamate wie Na- und Zn-Salze von Dialkyldithiocarbamaten, Tetramethylthiuramdisulfid, Kalium-N-methyl-dithiocarbamat;
Nitrile wie 2,4,5,6-Tetrachlorisophthalodinitril, Dinatrium-cyano-dithioimidocarbamat;
Chinoline wie 8-Hydroxychinolin und deren Cu-Salze;
Mucochlorsäure, 5-Hydroxy-2(5H)-furanon;
4,5-Dichlorodithiazolinon, 4,5-Benzdithiazolinon, 4,5-Trimethylendithiazolinon, 4,5-Dichlor-(3H)-1,2-dithiol-3-on, 3,5-Dimethyl-tetrahydro-1,3,5-thiadiazin-2-thion, N-(2-p-Chlorbenzoylethyl)-hexaminiumchlorid, Kalium-N-hydroxymethyl-N'-methyl-dithiocarbamat,
2-Oxo-2-(4-hydroxy-phenyl)acethydroximsäure-chlorid,
Phenyl-(2-chlor-cyan-vinyl)sulfon,
Phenyl-(1,2-dichlor-2-cyan-vinyl)sulfon;
Ag, Zn oder Cu-haltige Zeolithe allein oder eingeschlossen in polymere Wirkstoffe, oder auch Mischungen aus mehrern der oben genannten Fungizide.

Wie bereits erwähnt eignen sich die Wirkstoffmischungen bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorratsschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp.
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aieurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Phylloxera vastatrix, Pemphigus spp., Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp, Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die erfindungsgemäßen Wirkstoffmischungen können in ihren handelsüblichen Formulierungen sowie in den aus diesen Formulierungen bereiteten Anwendungsformen in Mischung mit anderen Wirkstoffen, wie. Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe. Im einzelnen seien die weiter obengenannten Insektizide und Fungizide als Zumischpartner genannt.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Wirkstoffmischungen können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxy-methylcellulose, natürliche und synthetische pulverige; körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffmischung, vorzugsweise zwischen 0,5 und 90 Gewichtsprozent Wirkstoffmischung.

## Patentansprüche

1. Verwendung von Mitteln enthaltend eine synergistisch wirksame Menge von Fipronil und mindestens eine Verbindung der Formel (I) 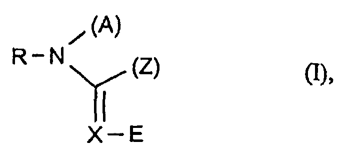 in welcher
R für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind, Hydroxy, Halogen, Cyano, Nitro, Amino, Monoalkyl- und Dialkylamino mit 1 bis 4 Kohlenstoffatomen je Alkylgruppe substituiertes Heteroarylmethyl mit bis zu 6 Ringatomen und N, O, S als Heteroatomen oder für Wasserstoff steht,
A für Wasserstoff oder Alkyl mit 1 bis 4 Kohlenstoffatomen steht,
E für NO₂ oder CN steht,
X für =CH- oder =N- steht,
Z für Alkyl oder für 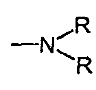 steht, und
A und Z gemeinsam mit den Atomen, an welche sie gebunden sind, einen gesättigten oder ungesättigten, 5- bis 7-gliedrigen heterocyclischen Ring bilden, der weitere 1 oder 2 gleiche oder verschiedene Heteroatome und/oder Heterogruppen enthalten kann, wobei als Heteroatome Sauerstoff, Schwefel oder Stickstoff und als Heterogruppen N-Alkyl in Frage kommen, wobei Alkyl der N-Alkylgruppe vorzugsweise 1 bis 4 Kohlenstoffatome enthält, zum Schutz von Holz, Holzprodukten und Holzwerkstoffen.

2. Verwendung gemäß Anspruch 1, **gekennzeichnet dadurch, dass** die Mittel Fipronil und mindestens eine der Verbindungen der folgenden Gruppe von Verbindungen 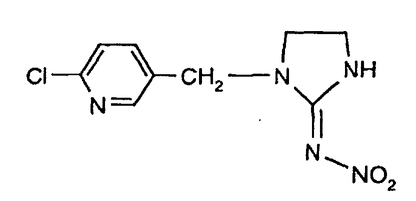 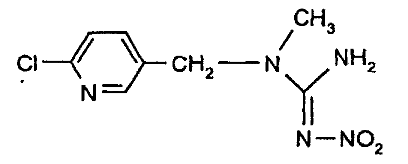 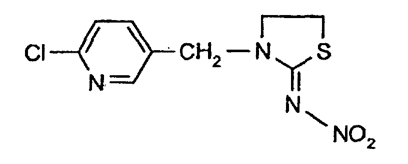 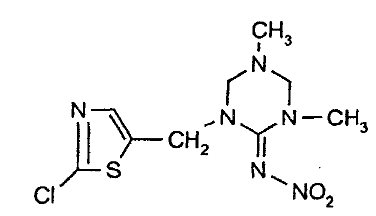 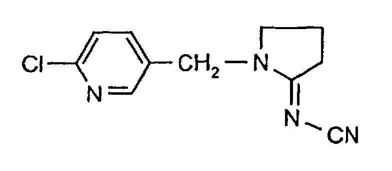 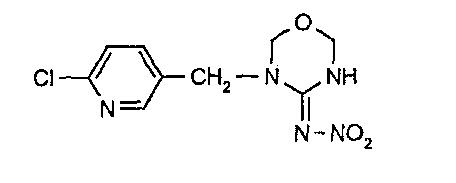 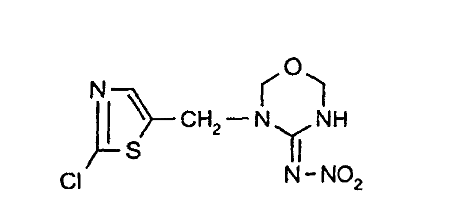 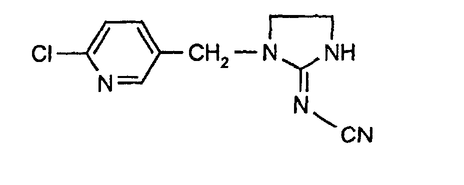 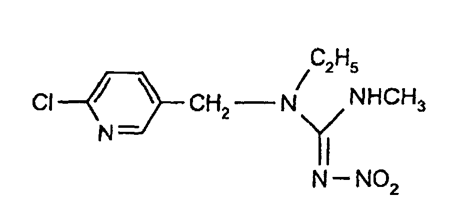 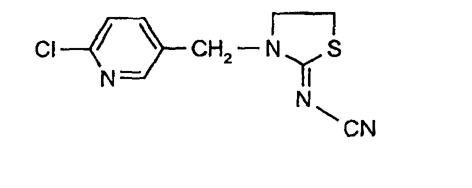 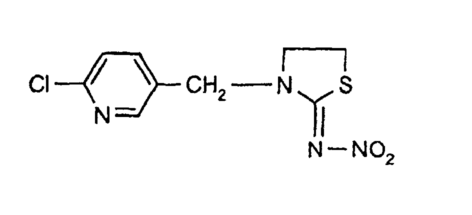 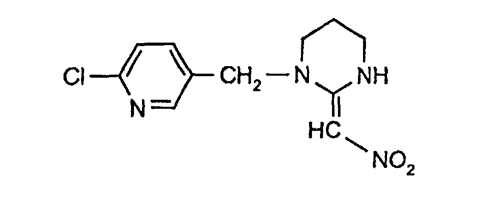 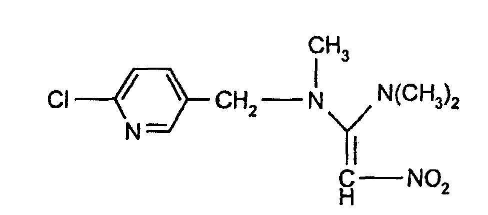 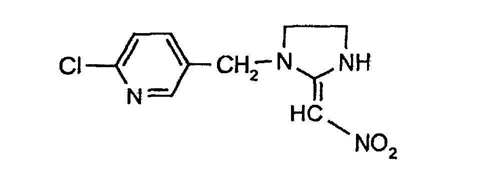 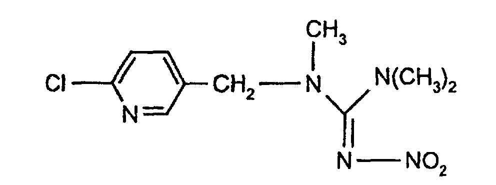 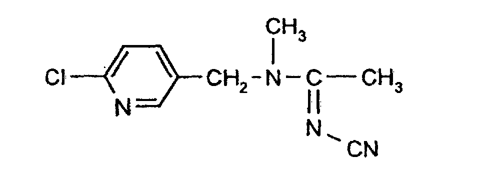 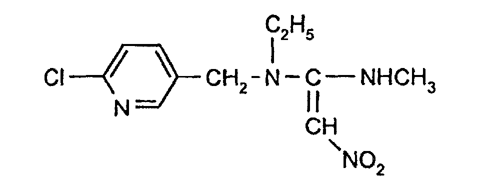 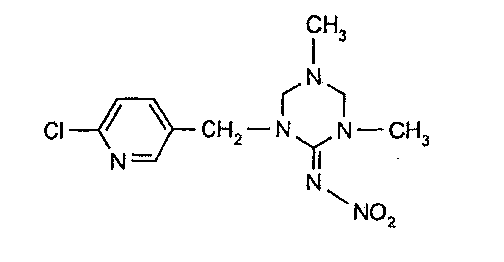 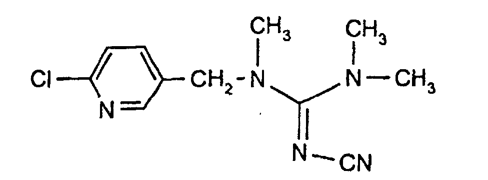 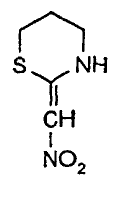 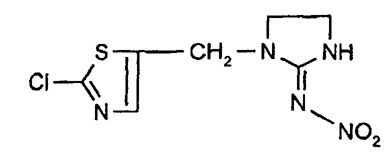 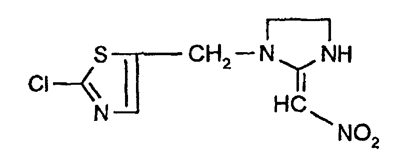 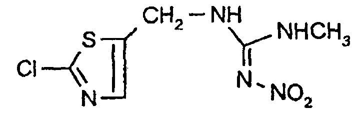 enthalten.

3. Verwendung gemäß Anspruch 1, **dadurch gekennzeichnet, dass** die Mittel Fipronil und mindestens eine Verbindung der Formel (IIa), (IIb), (IIc), (IId), (IIe), (IIf), (IIg), (IIh) oder (IIi) 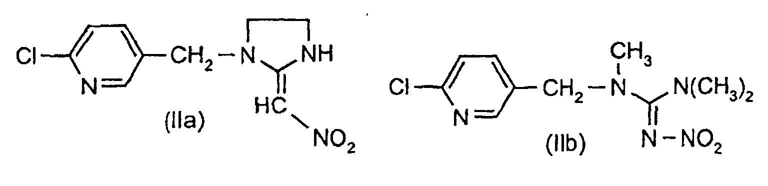 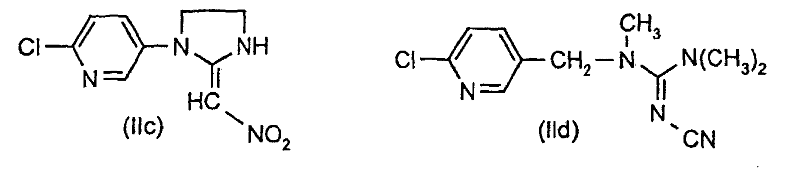 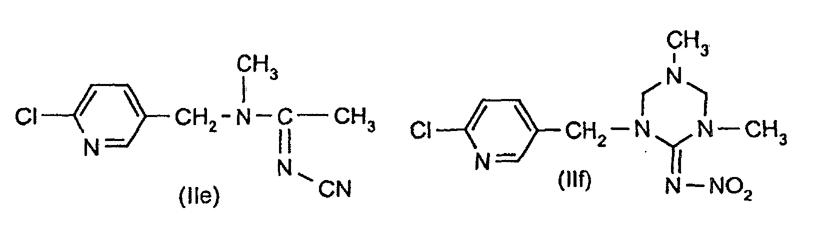 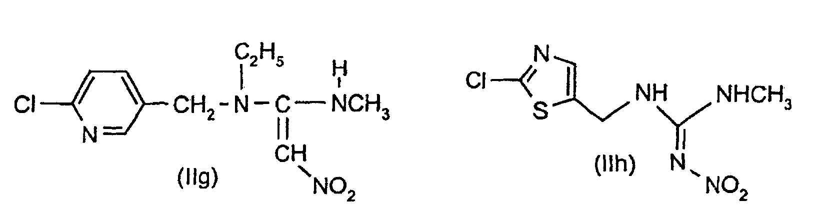 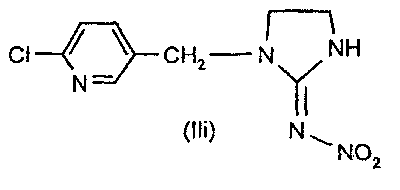 enthalten.

4. Verwendung gemäß Anspruch 1, **dadurch gekennzeichnet, dass** die Mittel Fipronil und mindestens eine der Verbindungen der Formel (I) in einem Verhältnis von 1:100 bis 100:1 enthalten.

5. Verfahren zum Schützen von Holz vor insketizidem Befall, **dadurch gekennzeichnet, dass** man Mittel enthaltend eine synergistisch wirksame Menge von Fipronil und mindestens eine Verbindung der Formel (I) 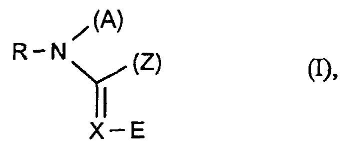 in welcher
R für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind, Hydroxy, Halogen, Cyano, Nitro, Amino, Monoalkyl- und Dialkylamino mit 1 bis 4 Kohlenstoffatomen je Alkylgruppe substituiertes Heteroarylmethyl mit bis zu 6 Ringatomen und N, O, S als Heteroatomen oder für Wasserstoff steht,
A für Wasserstoffoder Alkyl mit 1 bis 4 Kohlenstoffatomen steht,
E für NO₂ oder CN steht,
X für =CH- oder =N- steht,
Z für Alkyl oder für 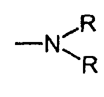 steht, und
A und Z gemeinsam mit den Atomen, an welche sie gebunden sind, einen gesättigten oder ungesättigten, 5- bis 7-gliedrigen heterocyclischen Ring bilden, der weitere 1 oder 2 gleiche oder verschiedene Heteroatome und/oder Heterogruppen enthalten kann, wobei als Heteroatome Sauerstoff, Schwefel oder Stickstoff und als Heterogruppen N-Alkyl in Frage kommen, wobei Alkyl der N-Alkylgruppe vorzugsweise 1 bis 4 Kohlenstoffatome enthält, auf Holz, Holzprodukte und Holzwerkstoffe aufträgt bzw. Holz, Holzprodukte und Holzwerkstoffe mit diesen Mitteln behandelt.

6. Verfahren gemäß Anspruch 5, **gekennzeichnet dadurch, dass** die Mittel Fipronil und mindestens eine der Verbindungen der folgenden Gruppe von Verbindungen 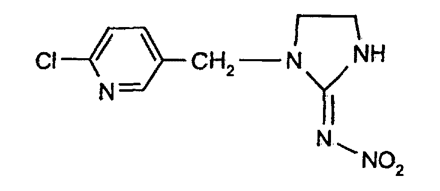 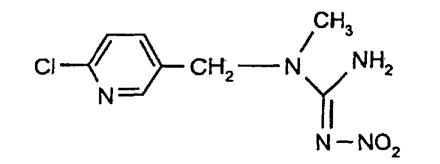 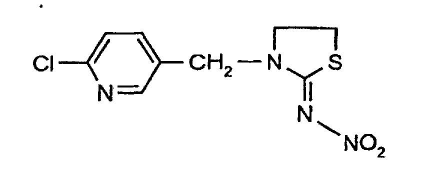 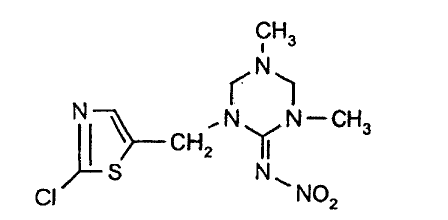 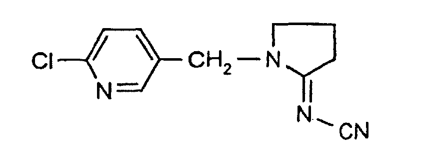 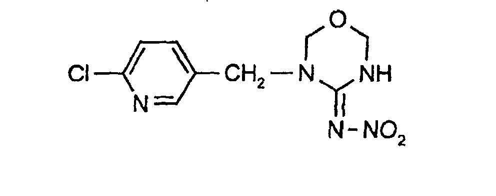 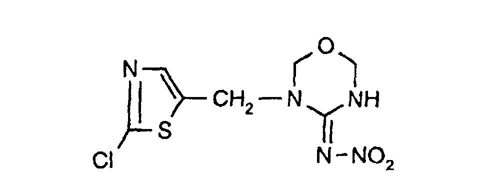 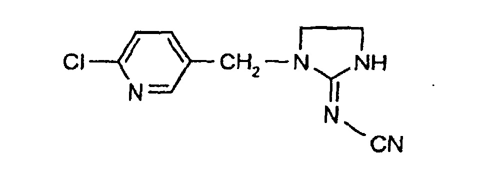 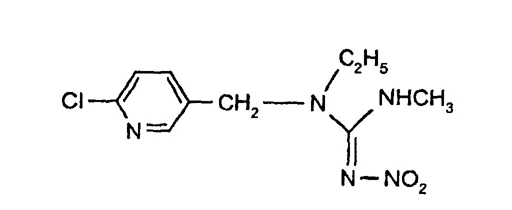 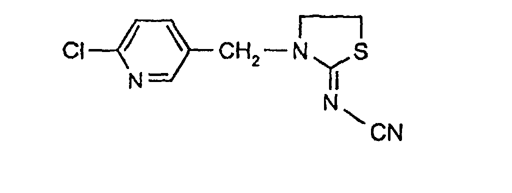 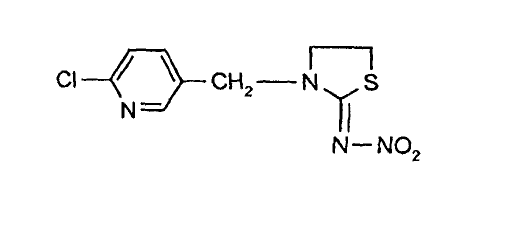 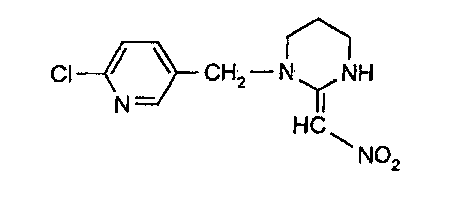 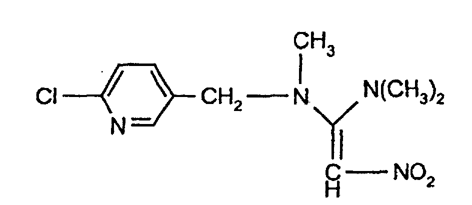 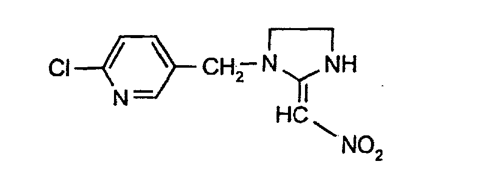 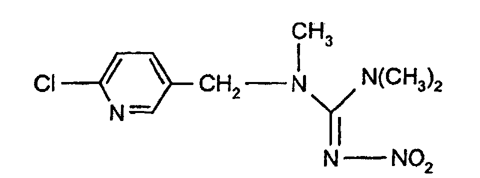 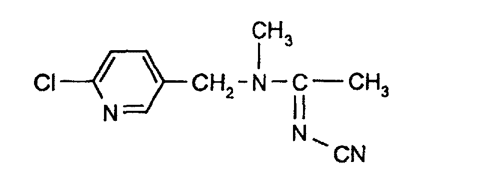 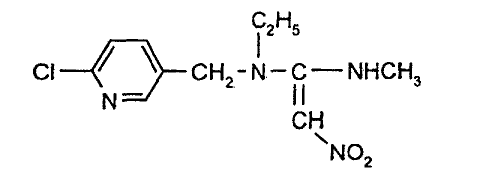 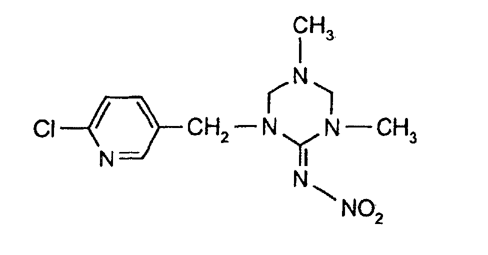 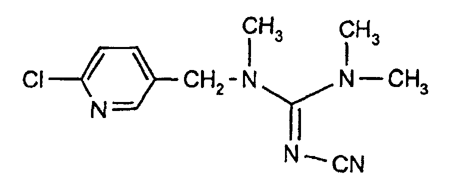 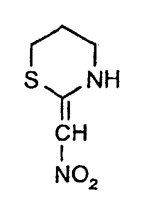 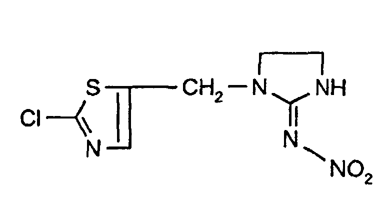 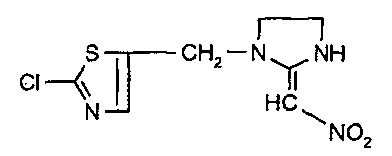 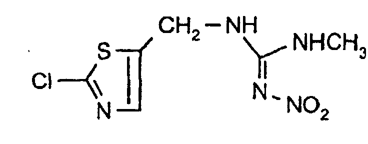 enthalten.

7. Verfahren gemäß Anspruch 1, **dadurch gekennzeichnet, dass** die Mittel Fipronil und mindestens eine Verbindung der Formel (IIa), (IIb), (IIc), (IId), (IIe), (IIf), IIg), (IIh) oder (IIi) 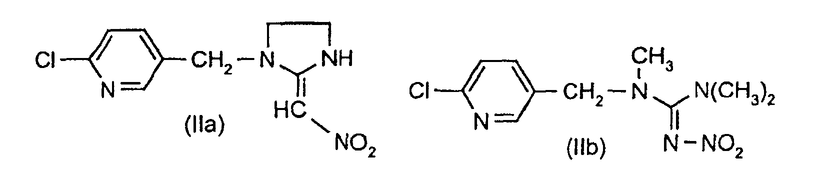 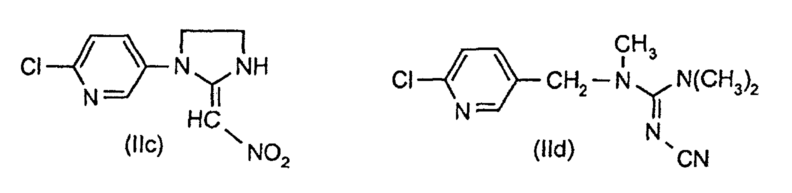 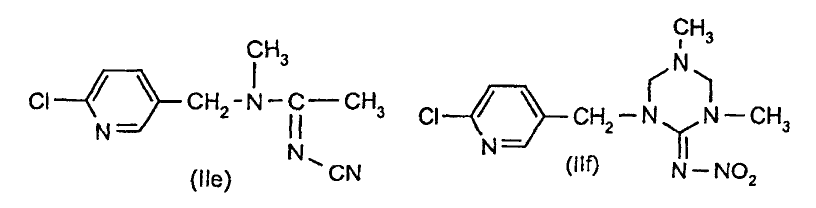 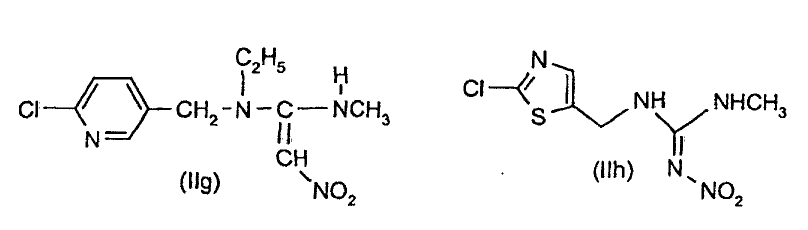 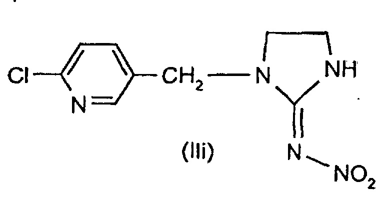 enthalten.

8. Verfahren gemäß Anspruch 1, **dadurch gekennzeichnet, dass** die Mittel Fipronil und mindestens eine der Verbindungen der Formel (I) in einem Verhältnis von 1:100 bis 100:1 enthalten.

9. Mittel zum Schutz von technischen Materialien enthaltend eine synergistisch wirksame Menge von Fipronil und mindestens eine Verbindung der Formel (I) 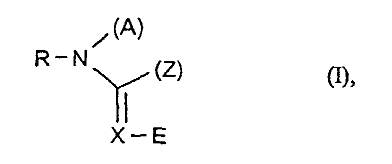 in welcher
R für gegebenenfalls durch Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 5 Halogenatomen, wobei die Halogenatome gleich oder verschieden sind, Hydroxy, Halogen, Cyano, Nitro, Amino, Monoalkyl- und Dialkylamino mit 1 bis 4 Kohlenstoffatomen je Alkylgruppe substituiertes Heteroarylmethyl mit bis zu 6 Ringatomen und N, O, S als Heteroatomen oder für Wasserstoff steht,
A für Wasserstoff oder Alkyl mit 1 bis 4 Kohlenstoffatomen steht,
E für NO₂ oder CN steht,
X für =CH- oder =N- steht,
Z für Alkyl oder für 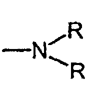 steht, und
A und Z gemeinsam mit den Atomen, an welche sie gebunden sind, einen gesättigten oder ungesättigten, 5- bis 7-gliedrigen heterocyclischen Ring bilden, der weitere 1 oder 2 gleiche oder verschiedene Heteroatome und/oder Heterogruppen enthalten kann, wobei als Heteroatome Sauerstoff, Schwefel oder Stickstoff und als Heterogruppen N-Alkyl in Frage kommen, wobei Alkyl der N-Alkylgruppe vorzugsweise 1 bis 4 Kohlenstoffatome enthält, mit ausnahme der Verbindungen (E)-N¹-((6-chlor-3-pyridyl)methyl)-N²-cyano-N¹-methyl-acetamidine (Acetamiprid), (E)-N-((6-chlor-3-pyridyl)methyl)-N-ethyl-N'-methyl-2-nitrovinylidendiamin (Nitenpyram) und 1-(6-Chlor-3-pyridylmethyl)-N-nitroimidazolidin-2-ylidenamin (Iidachloprid).

10. Mittel gemäß Anspruch 1, enthaltend Fipronil und mindestens eine der Verbindungen der folgenden Gruppe von Verbindungen

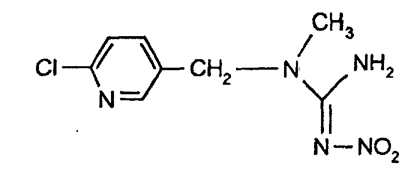

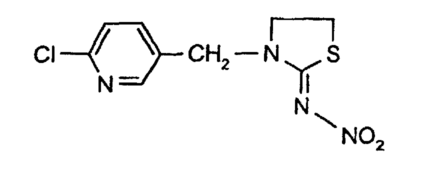

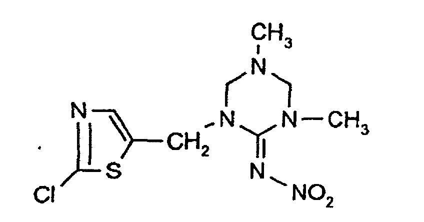

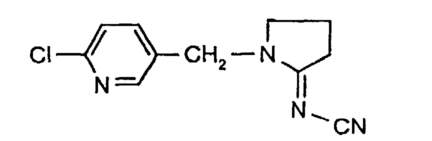

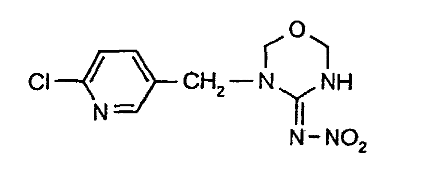

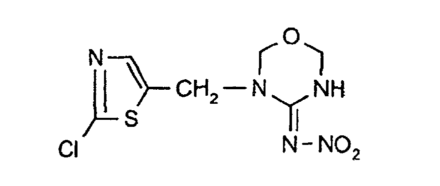

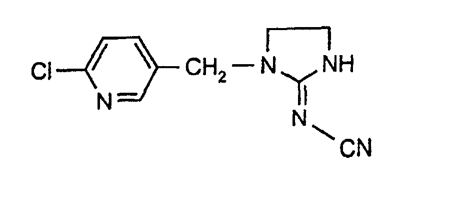

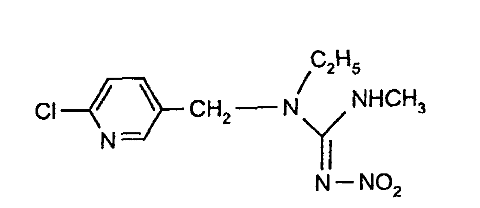

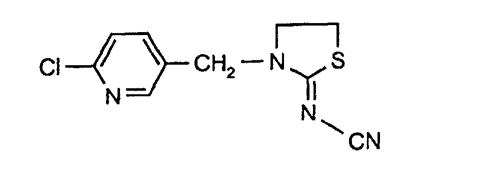

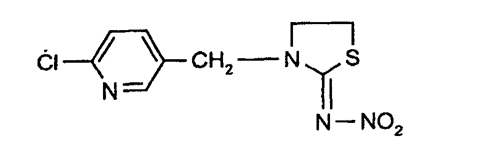

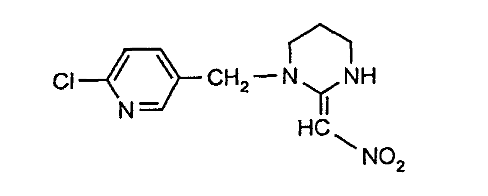

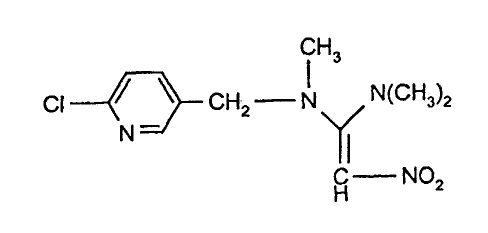

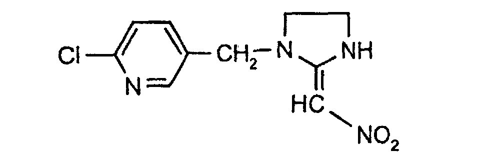

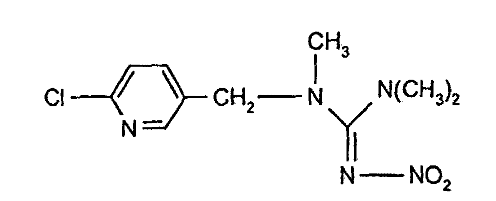

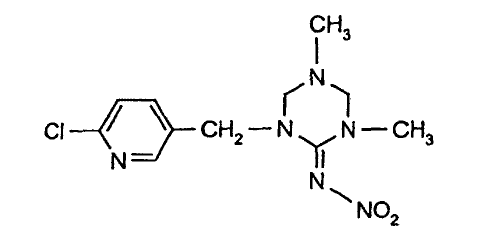

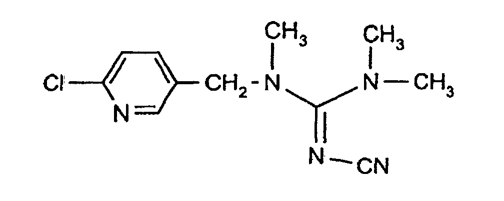

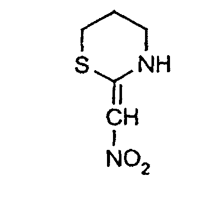

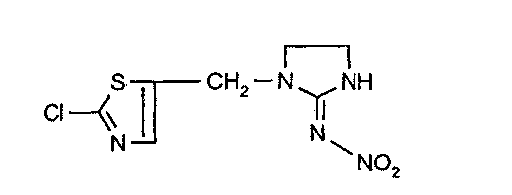

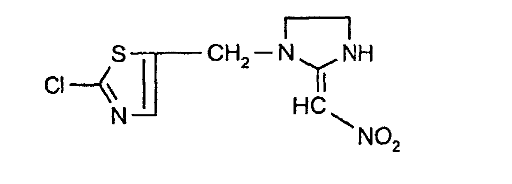

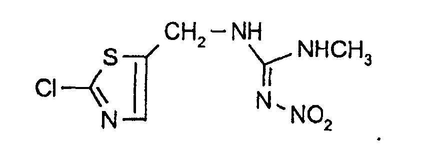

11. Mittel gemäß Anspruch 1, enthaltend Fipronil und mindestens eine Verbindung der Formel (IIa), (IIb), (IIc), (IId), (IIf) oder (IIh)

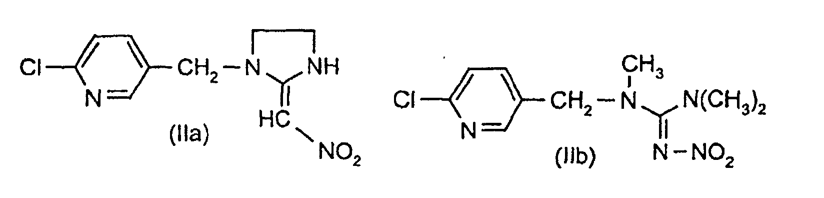

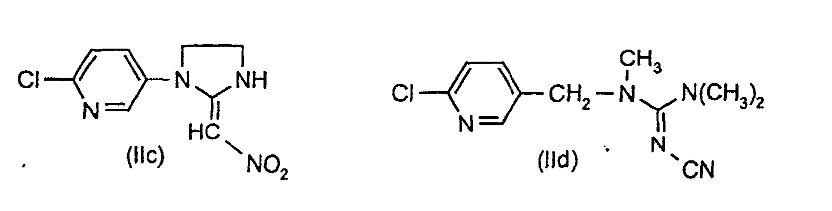

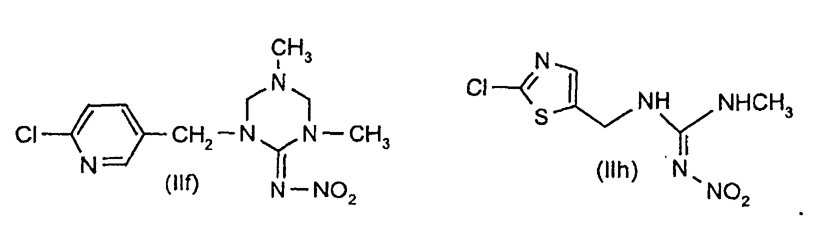

12. Mittel gemäß Anspruch 9, enthaltend Fipronil und mindestens eine der Verbindungen der Formel (I) in einem Verhältnis von 1:100 bis 100:1.

13. Verfahren zur Herstellung von Holzschutzmitteln, **dadurch gekennzeichnet, dass** man Mittel gemäß einem der Ansprüche 9 bis 12 mit üblichen Holzschutzmittelbestandteilen und gegebenenfalls weiteren Wirkstoffen vermischt.

14. Verwendung eines Mittels enthaltend eine synergistisch wirksame Menge Fipronil und die Verbindung der Formel 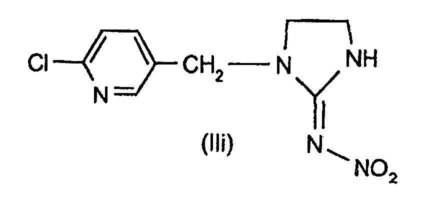 zum Schutz von Holz, Holzprodukten und Holzwerkstoffen.

## Claims

1. Use of compositions comprising a synergistically effective amount of fipronil and at least one compound of the formula (I) 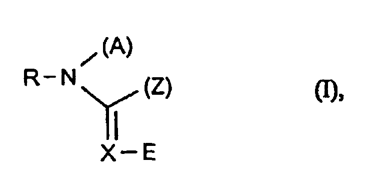 in which
R represents heteroarylmethyl having up to 6 ring atoms and N, O, S heteroatoms, which is optionally substituted by alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms, where the halogen atoms are identical or different, hydroxyl, halogen, cyano, nitro, amino, monoalkyl- and dialkylamino having 1 to 4 carbon atoms per alkyl group, or represents hydrogen,
A represents hydrogen or alkyl having 1 to 4 carbon atoms,
E represents NO₂ or CN,
X represents =CH- or =N-,
Z represents alkyl or 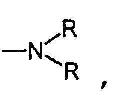 and
A and Z together with the atoms to which they are attached form a saturated or unsaturated 5- to 7-membered heterocyclic ring which may contain 1 or 2 identical or different heteroatoms and/or hetero groups, suitable heteroatoms being oxygen, sulphur or nitrogen and suitable hetero groups being N-alkyl, where the alkyl of the N-alkyl group preferably contains 1 to 4 carbon atoms, for protecting wood, wood products and timber.

2. Use according to Claim 1, **characterized in that** the compositions comprise fipronil and at least one of the compounds of the following group of compounds

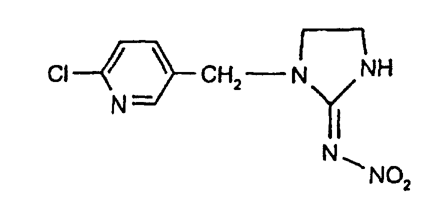

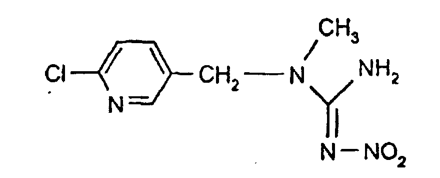

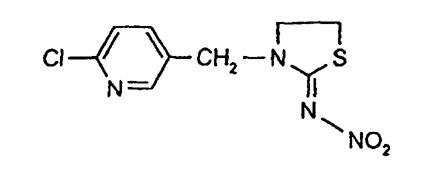

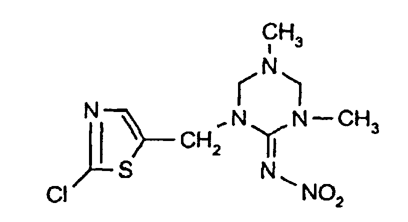

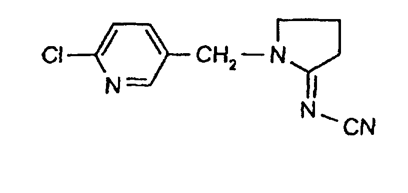

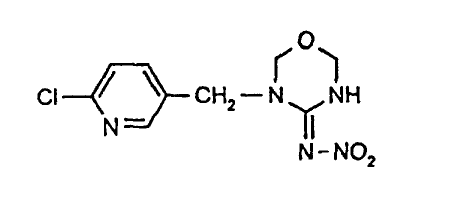

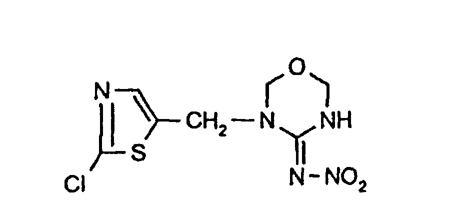

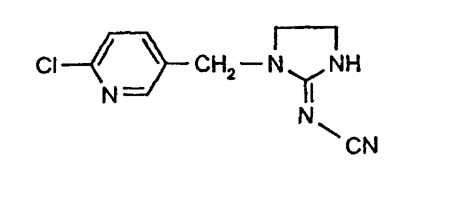

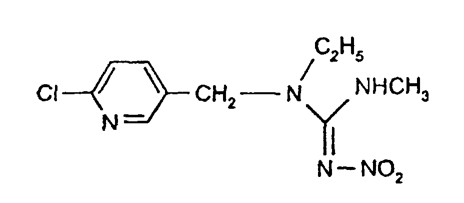

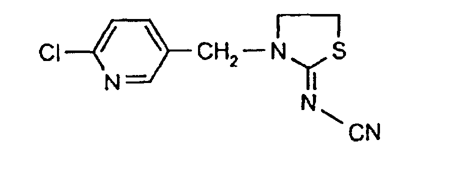

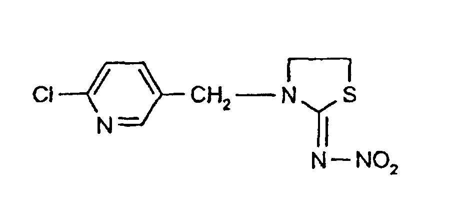

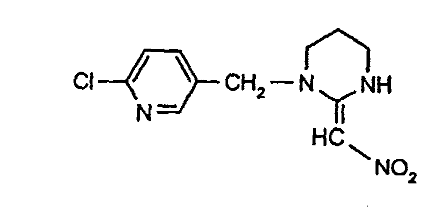

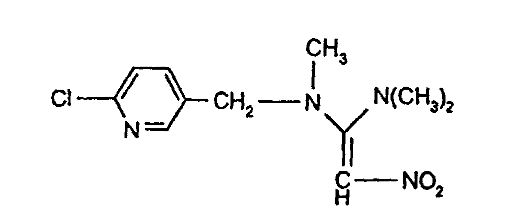

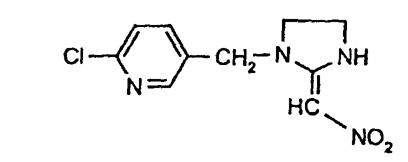

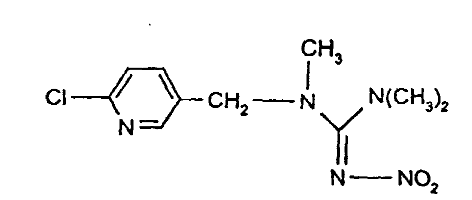

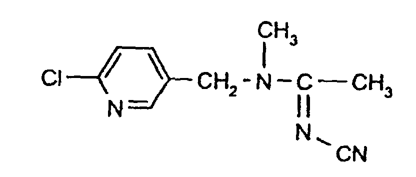

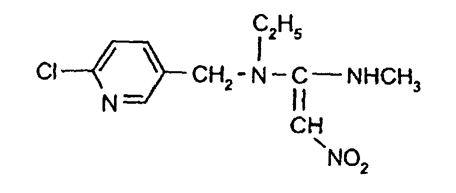

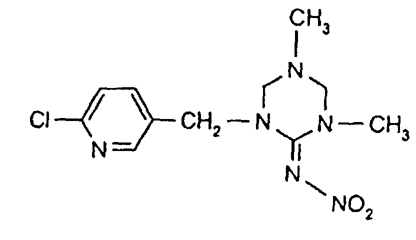

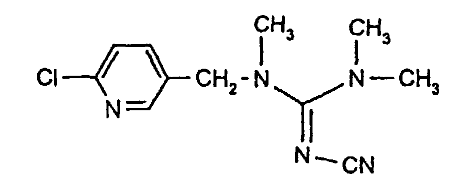

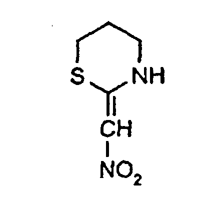

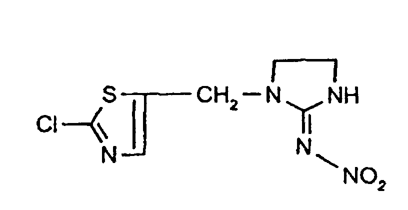

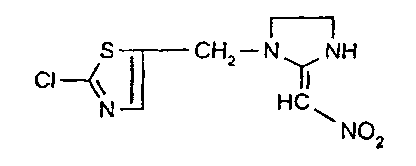

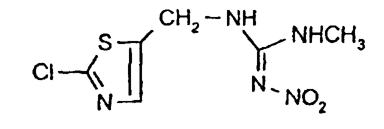

3. Use according to Claim 1, **characterized in that** the compositions comprise fipronil and at least one compound of the formula (IIa), (IIb), (IIc), (IId), (IIe), (IIf), (IIg), (IIh) or (IIi)

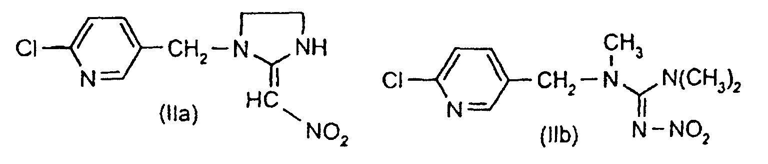

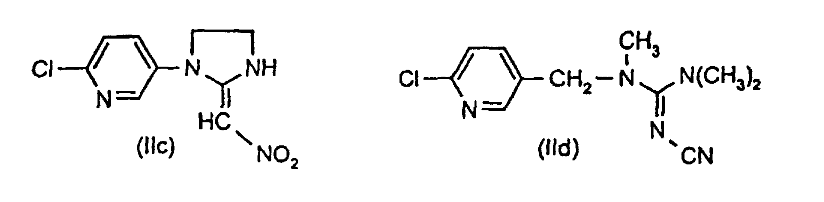

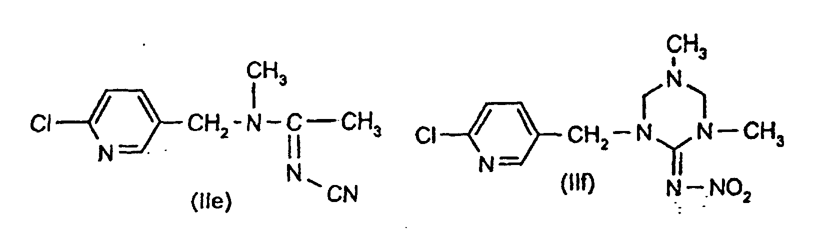

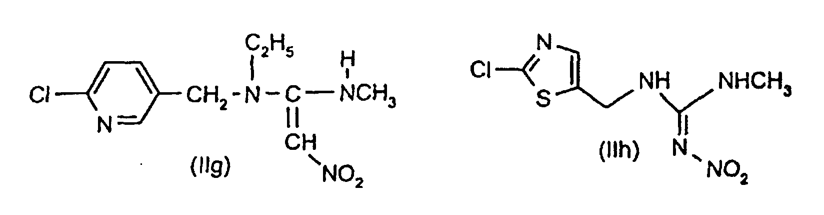

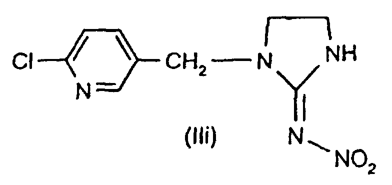

4. Use according to Claim 1, **characterized in that** the compositions comprise fipronil and at least one of the compounds of the formula (I) in a ratio of from 1:100 to 100:1.

5. Process for protecting wood against attack by insects, **characterized in that** compositions comprising a synergistically effective amount of fipronil and at least one compound of the formula (I) 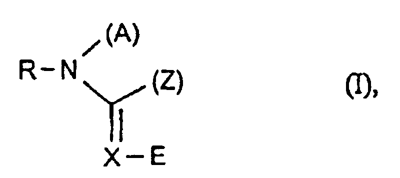 in which
R represents heteroarylmethyl having up to 6 ring atoms and N, O, S heteroatoms, which is optionally substituted by alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms, where the halogen atoms are identical or different, hydroxyl, halogen, cyano, nitro, amino, monoalkyl- and dialkylamino having 1 to 4 carbon atoms per alkyl group, or represents hydrogen,
A represents hydrogen or alkyl having 1 to 4 carbon atoms,
E represents NO₂ or CN,
X represents =CH- or =N-,
Z represents alkyl or 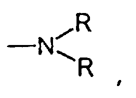 and
A and Z together with the atoms to which they are attached form a saturated or unsaturated 5- to 7-membered heterocyclic ring which may contain 1 or 2 identical or different heteroatoms and/or hetero groups, suitable heteroatoms being oxygen, sulphur or nitrogen and suitable hetero groups being N-alkyl, where the alkyl of the N-alkyl group preferably contains 1 to 4 carbon atoms, are applied to wood, wood products and timber, and/or wood, wood products and timber are treated with these compositions.

6. Process according to Claim 5, **characterized in that** the compositions comprise fipronil and at least one of the compounds of the following group of compounds

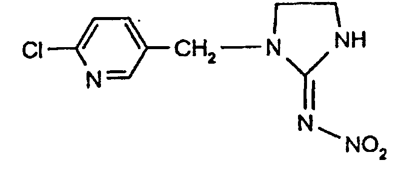

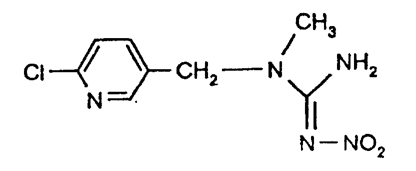

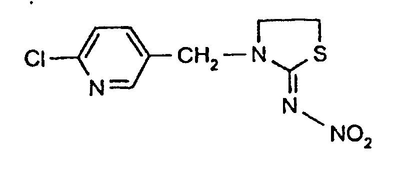

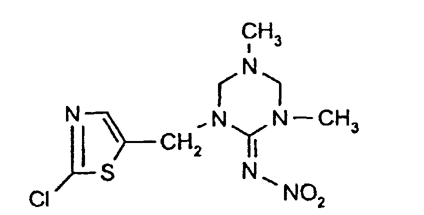

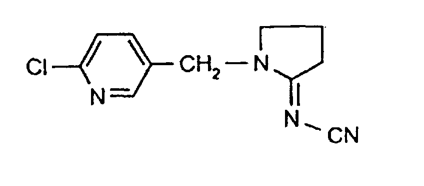

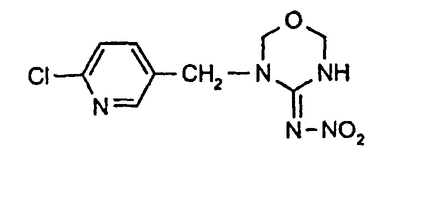

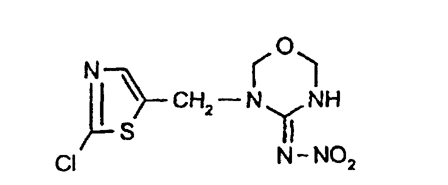

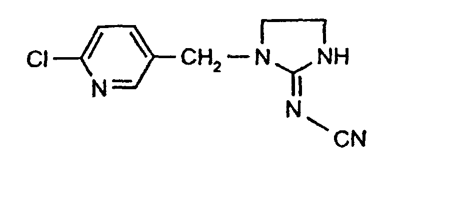

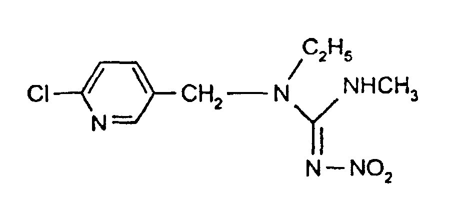

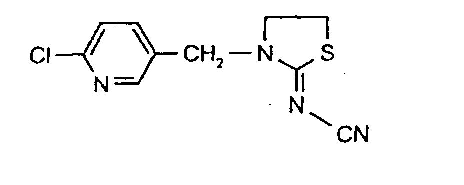

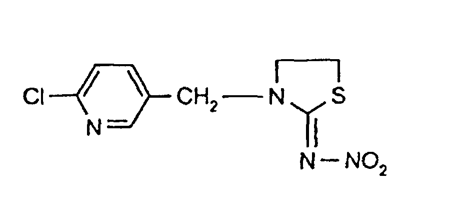

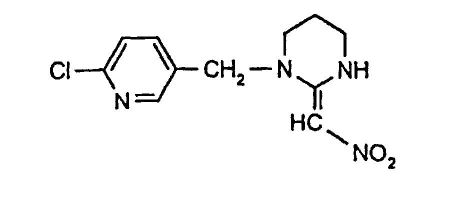

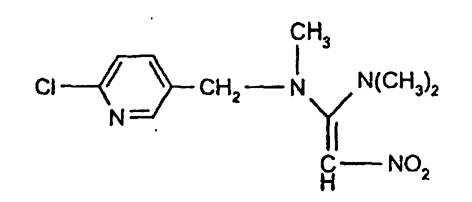

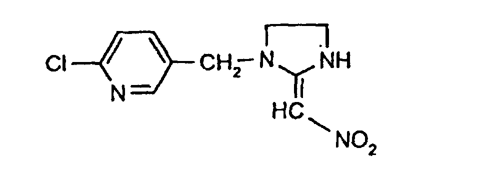

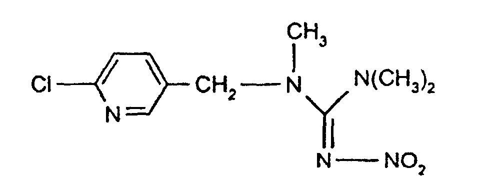

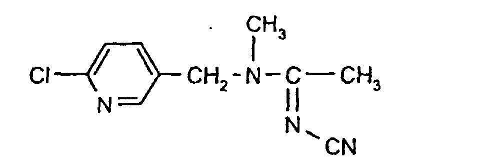

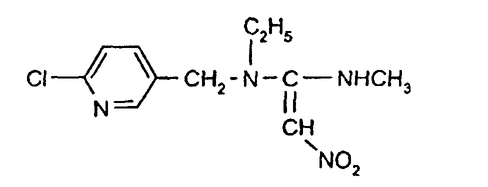

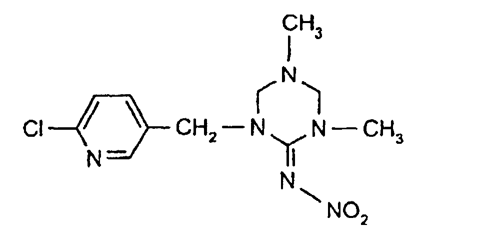

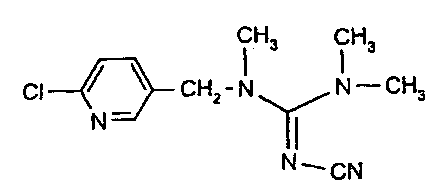

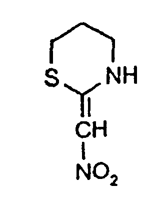

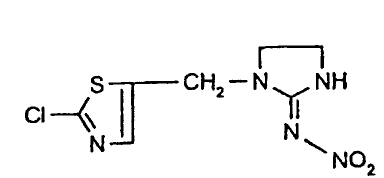

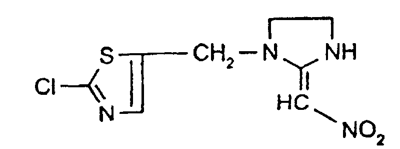

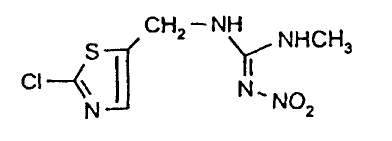

7. Process according to Claim 5, **characterized in that** the compositions comprise fipronil and at least one compound of the formula (IIa), (IIb), (IIc), (IId), (IIe), (IIf), (IIg), (IIh) or (IIi)

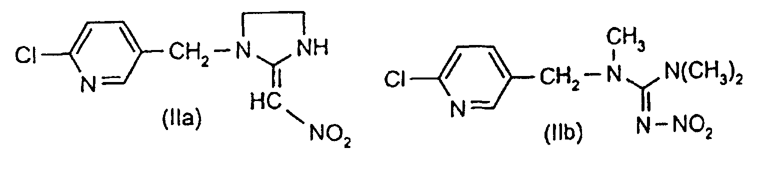

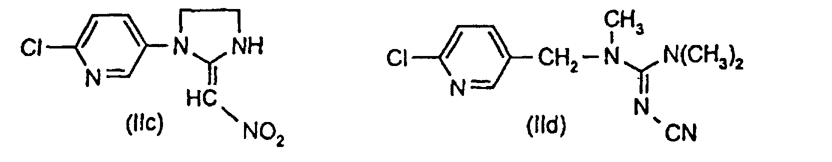

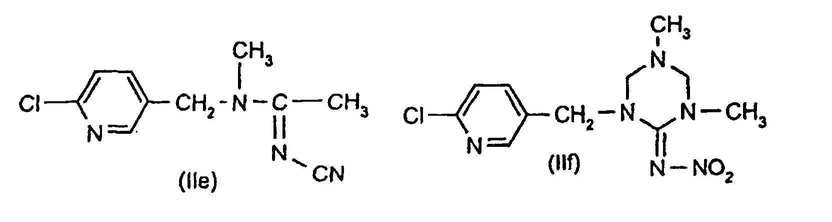

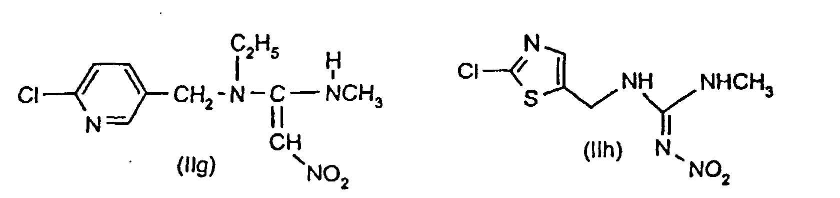

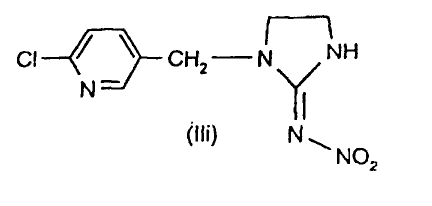

8. Process according to Claim 5, **characterized in that** the compositions comprise fipronil and at least one of the compounds of the formula (I) in a ratio of from 1:100 to 100:1.

9. Composition for protecting industrial materials, comprising a synergistically effective amount of fipronil and at least one compound of the formula (I) 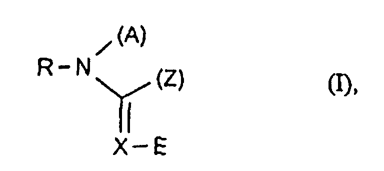 in which
R represents heteroarylmethyl having up to 6 ring atoms and N, O, S heteroatoms, which is optionally substituted by alkyl having 1 to 4 carbon atoms, alkoxy having 1 to 4 carbon atoms, alkylthio having 1 to 4 carbon atoms, halogenoalkyl having 1 to 4 carbon atoms and 1 to 5 halogen atoms, where the halogen atoms are identical or different, hydroxyl, halogen, cyano, nitro, amino, monoalkyl- and dialkylamino having 1 to 4 carbon atoms per alkyl group, or represents hydrogen,
A represents hydrogen or alkyl having 1 to 4 carbon atoms,
E represents NO₂ or CN,
X represents =CH- or =N-,
Z represents alkyl or 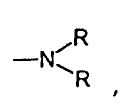 and
A and Z together with the atoms to which they are attached form a saturated or unsaturated 5- to 7-membered heterocyclic ring which may contain 1 or 2 identical or different heteroatoms and/or hetero groups, suitable heteroatoms being oxygen, sulphur or nitrogen and suitable hetero groups being N-alkyl, where the alkyl of the N-alkyl group preferably contains 1 to 4 carbon atoms, except for the compounds (E)-N¹-((6-chloro-3-pyridyl)methyl)-N²-cyano-N¹-methylacetamidine (acetamiprid), (E)-N-((6-chloro-3-3-pyridyl)methyl)-ethyl-N'-methyl-2-nitrovinylidenediamine (nitenpyram) and 1-(6-chloro-3-pyridylmethyl)-N-nitroimidazolidin-2-ylideneamine (imidacloprid).

10. Compositions according to Claim 9, comprising fipronil and at least one of the compounds of the following group of compounds

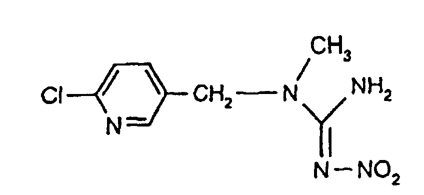

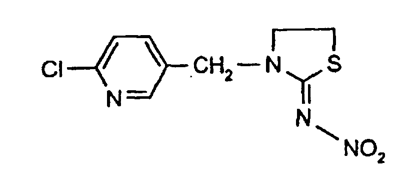

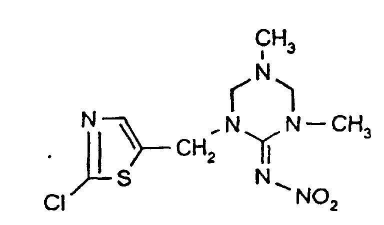

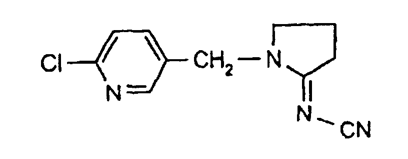

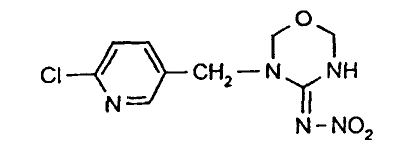

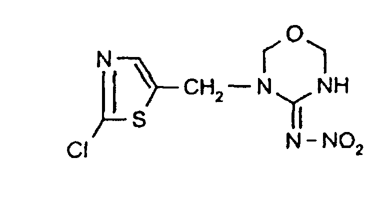

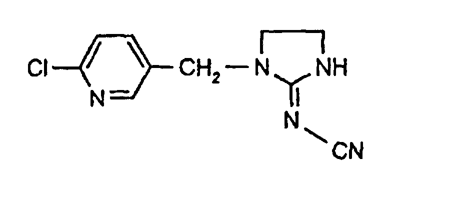

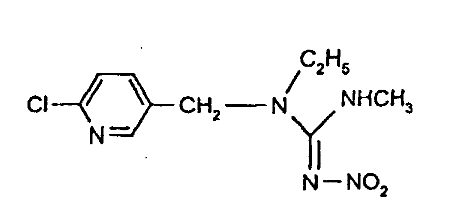

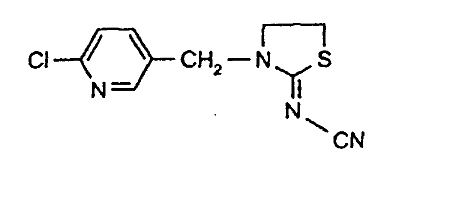

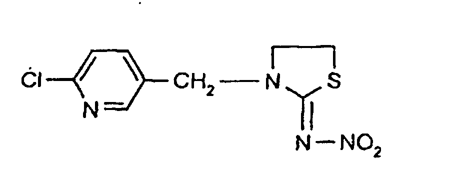

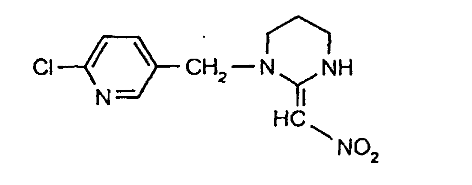

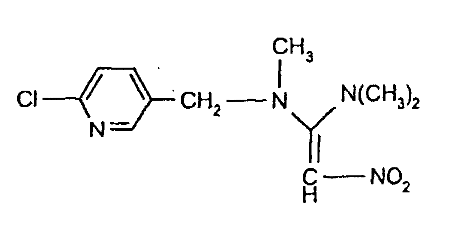

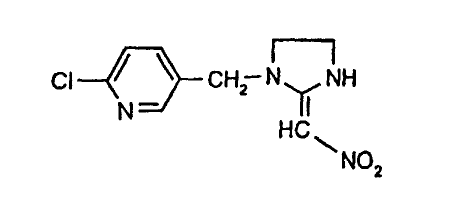

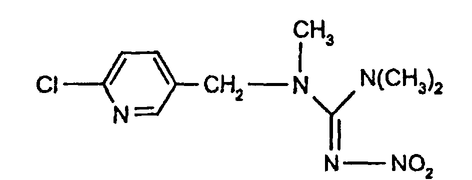

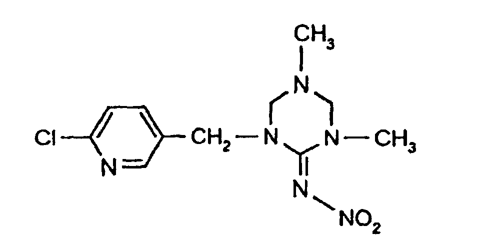

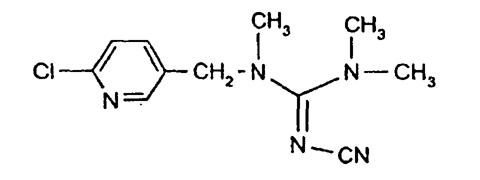

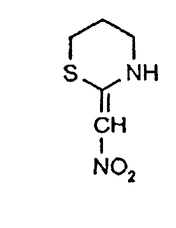

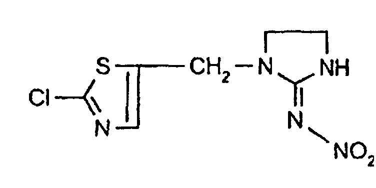

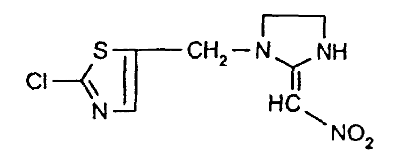

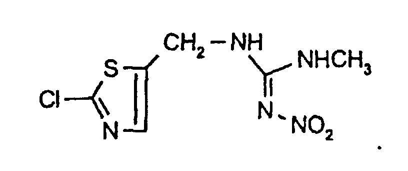

11. Compositions according to Claim 9, comprising fipronil and at least one of the compounds of the formula (IIa), (IIb), (IIc), (IId), (IIf) or (IIh)

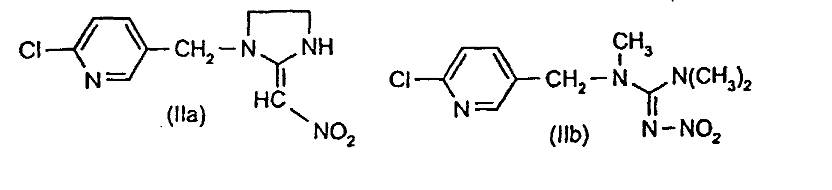

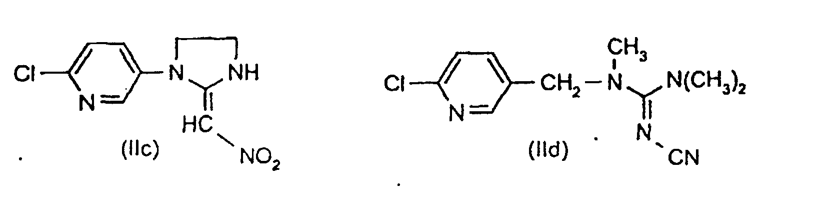

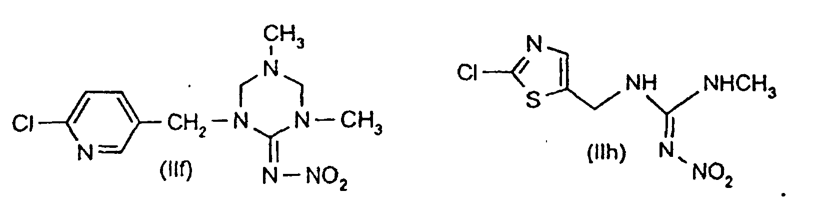

12. Compositions according to Claim 9, comprising fipronil and at least one of the compounds of the formula (I) in a ratio of from 1:100 to 100:1.

13. Process for preparing composition for the protection of wood, **characterized in that** compositions according to any of Claims 9 to 12 are mixed with customary components of compositions for protecting wood and, if appropriate, with further active compounds.

14. Use of a composition comprising a synergistically effective amount of fipronil and the compound of the formula 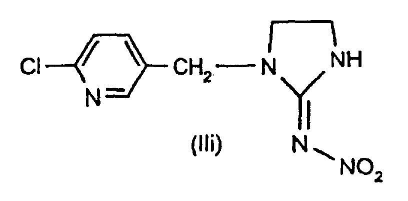 for protecting wood, wood products and timber.

## Revendications

1. Utilisation d'agents contenant une quantité à effet synergique de fipronil et d'au moins un composé de formule (I) 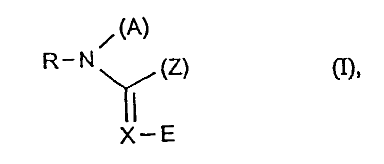 dans laquelle
R désigne un groupe hétéroarylméthyle ayant jusqu'à 6 atomes dans le noyau et N, O, S comme hétéroatomes, portant éventuellement un substituant alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes identiques ou différents, hydroxy, halogéno, cyano, nitro, amino, mono-alkyl- et dialkylamino ayant 1 à 4 atomes de carbone par groupe alkyle, ou représente l'hydrogène,
A est l'hydrogène ou un groupe alkyle ayant 1 à 4 atomes de carbone,
E est un groupe NO₂ ou CN,
X est un groupe =CH- ou =N-,
Z est un groupe alkyle ou un groupe 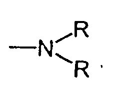 et
A et Z forment conjointement avec les atomes auxquels ils sont liés un noyau hétérocyclique pentagonal à heptagonal saturé ou non saturé, qui peut encore contenir 1 ou 2 hétéroatomes et/ou hétérogroupes identiques ou différents, en considérant comme hétéroatomes l'oxygène, le soufre ou l'azote et comme hétérogroupes des groupes N-alkyle, le radical alkyle d'un groupe N-alkyle contenant de préférence 1 à 4 atomes de carbone, pour la protection de bois, de produits à base de bois et de matériaux à base de bois.

2. Utilisation suivant la revendication 1, **caractérisée en ce que** les agents contiennent du fipronil et au moins l'un des composés du groupe de composés suivant : 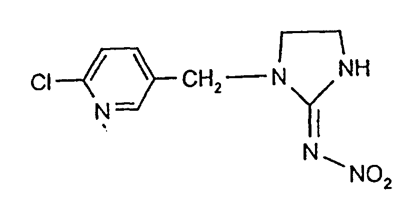 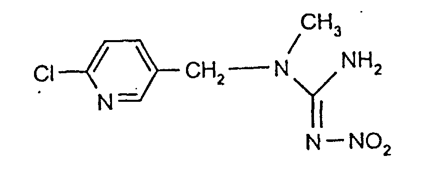 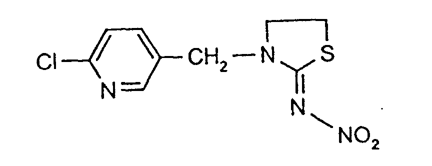 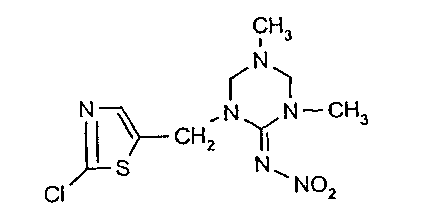 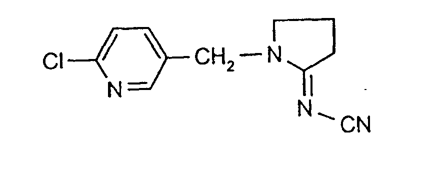 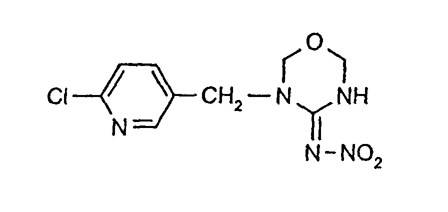 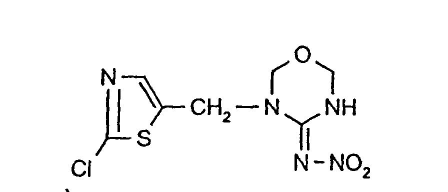 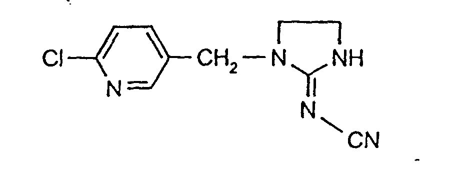 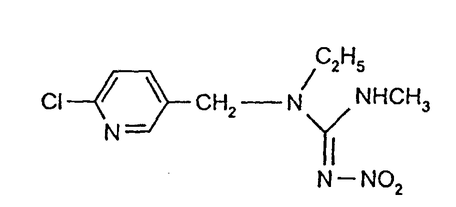 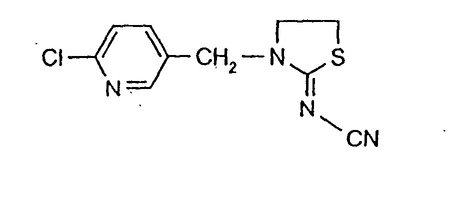 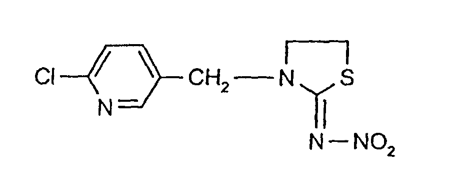 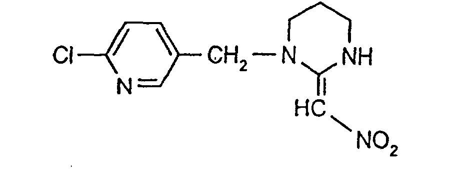 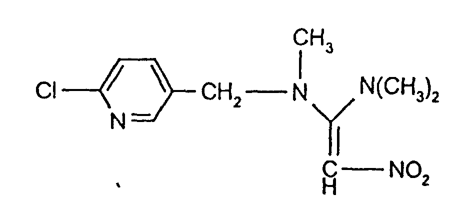 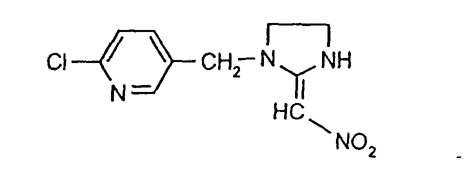 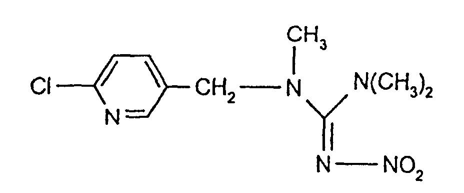 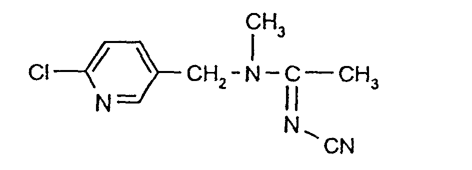 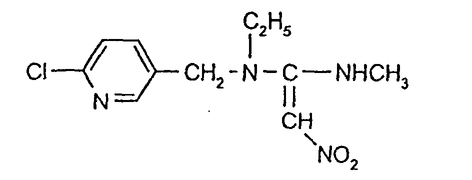 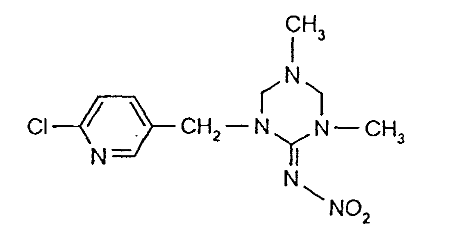 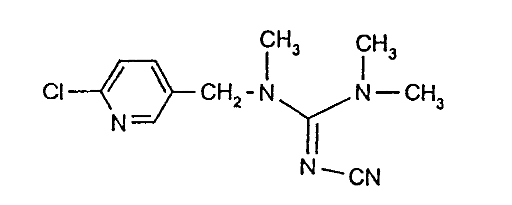 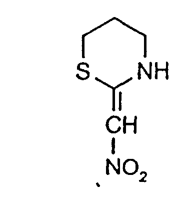 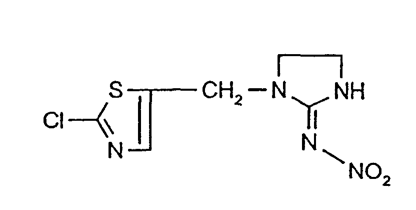 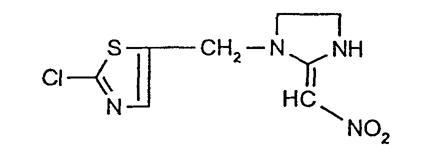 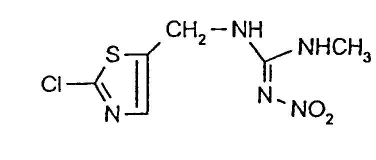 enthalten.

3. Utilisation suivant la revendication 1, **caractérisée en ce que** les agents contiennent du fipronil et au moins un composé de formule (IIa), (IIb), (IIc), (IId), (IIe), (IIf), (IIg), (IIh) ou (IIi)

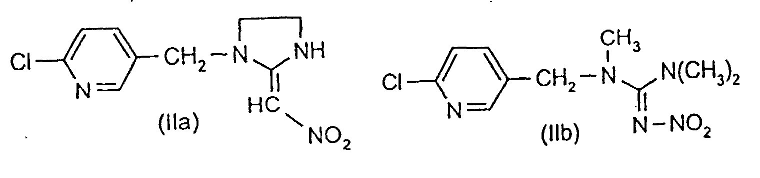

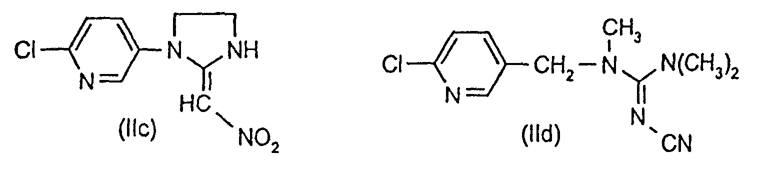

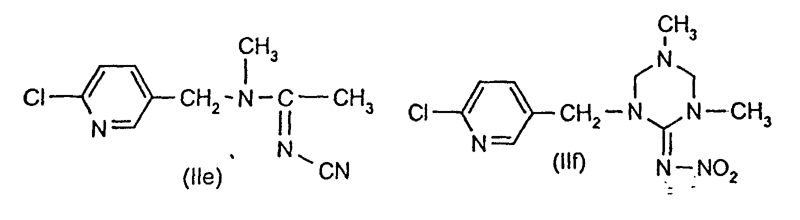

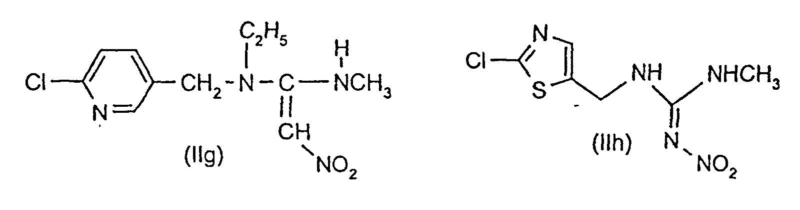

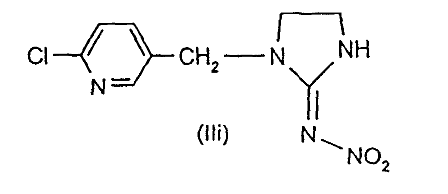

4. Utilisation suivant la revendication 1, **caractérisée en ce que** les agents contiennent du fipronil et au moins l'un des composés de formule (I) dans un rapport de 1:100 à 100:1.

5. Procédé pour protéger du bois d'une attaque par des insectes, **caractérisé en ce qu'**on applique des agents contenant une quantité à effet synergique de fipronil et d'au moins un composé de formule (I) 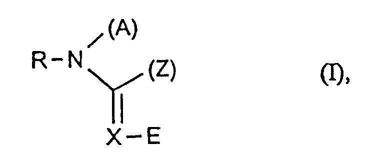 dans laquelle
R désigne un groupe hétéroarylméthyle ayant jusqu'à 6 atomes dans le noyau et N, O, S comme hétéroatomes, portant éventuellement un substituant alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes identiques ou différents, hydroxy, halogéno, cyano, nitro, amino, mono-alkyl- et dialkylamino ayant 1 à 4 atomes de carbone par groupe alkyle, ou représente l'hydrogène,
A est l'hydrogène ou un groupe alkyle ayant 1 à 4 atomes de carbone,
E est un groupe NO₂ ou CN,
X est un groupe =CH- ou =N-,
Z est un groupe alkyle ou un groupe 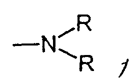 et
A et Z forment conjointement avec les atomes auxquels ils sont liés un noyau hétérocyclique pentagonal à heptagonal saturé ou non saturé, qui peut encore contenir 1 ou 2 hétéroatomes et/ou hétérogroupes identiques ou différents, en considérant comme hétéroatomes l'oxygène, le soufre ou l'azote et comme hétérogroupes des groupes N-alkyle, le radical alkyle d'un groupe N-alkyle contenant de préférence 1 à 4 atomes de carbone, sur du bois, des produits à base de bois et des matériaux à base de bois, c'est-à-dire qu'on traite du bois, des produits à base de bois et des matériaux à base de bois avec ces compositions.

6. Procédé suivant la revendication 5, **caractérisé en ce que** les agents contiennent du fipronil et au moins l'un des composés du groupe de composés suivant :

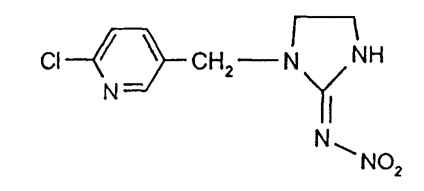

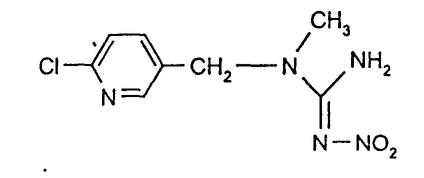

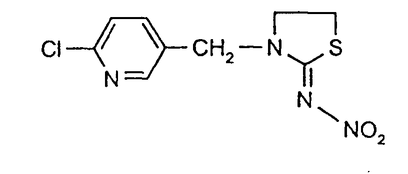

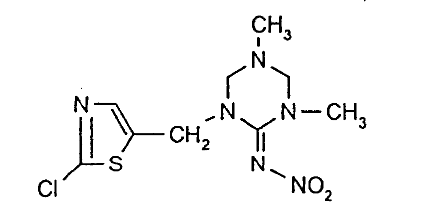

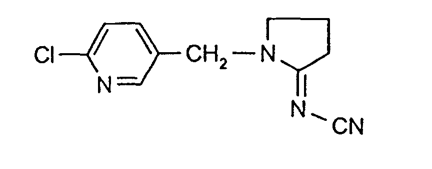

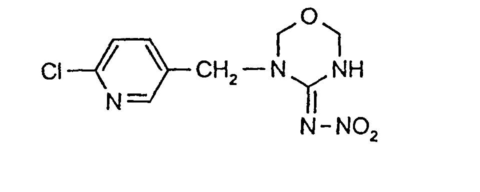

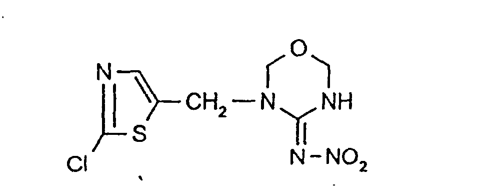

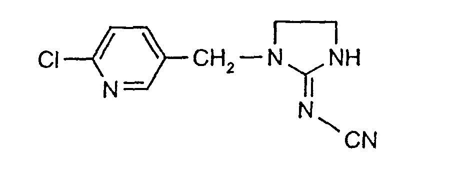

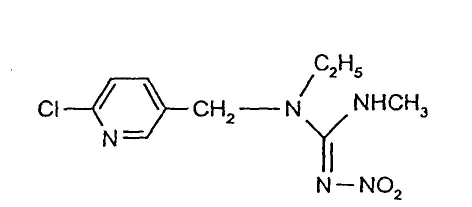

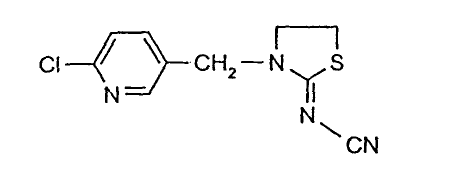

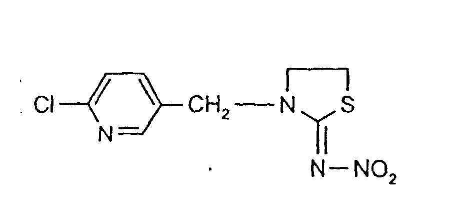

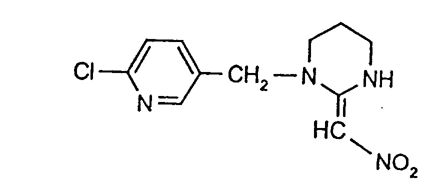

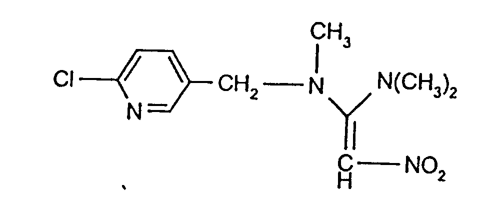

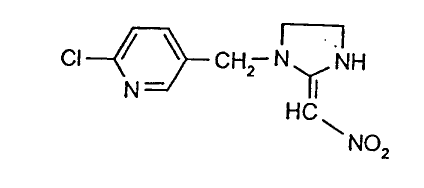

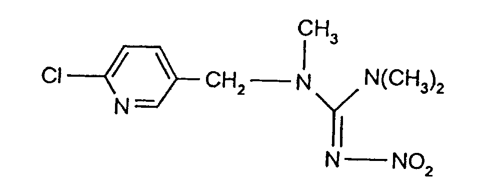

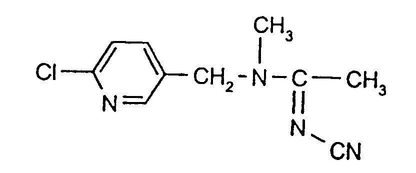

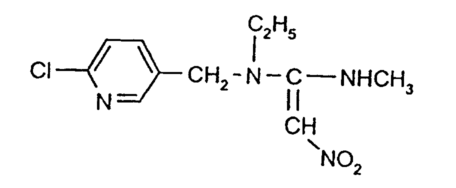

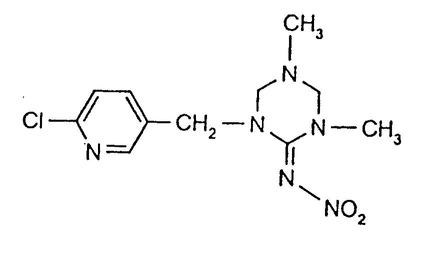

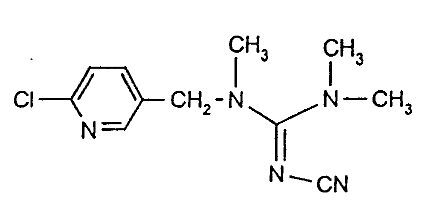

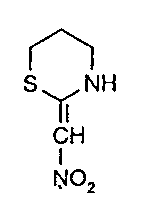

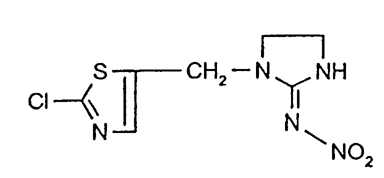

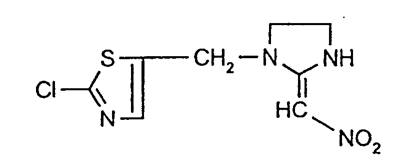

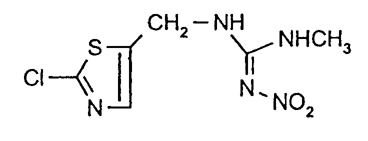

7. Procédé suivant la revendication 1, **caractérisé en ce que** les agents contiennent du fipronil et au moins un composé de formule (IIa), (IIb), (IIc), (IId), (IIe), (IIf), (IIg), (IIh) ou (IIi)

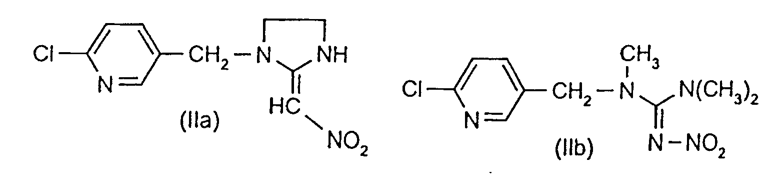

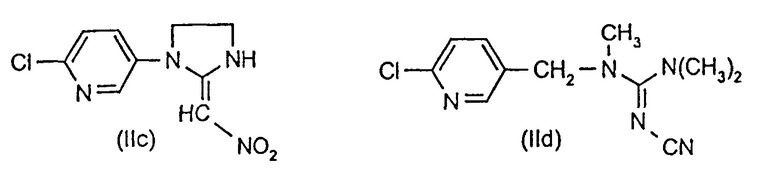

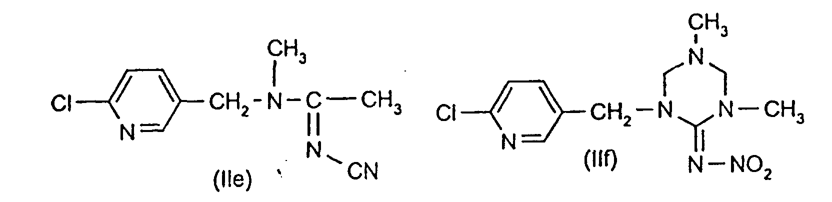

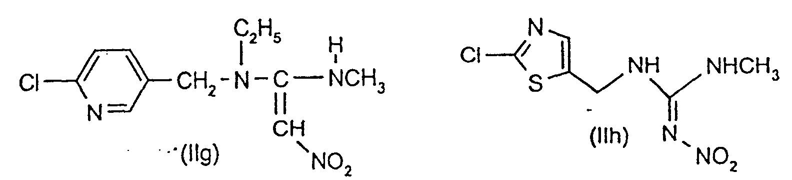

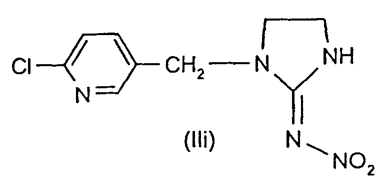

8. Procédé suivant la revendication 1, **caractérisé en ce que** les agents contiennent du fipronil et l'un au moins des composés de formule (I) dans un rapport de 1:100 à 100:1.

9. Agent destiné au traitement de matériaux techniques, contenant une quantité à effet synergique de fipronil et d'au moins un composé de formule (I) 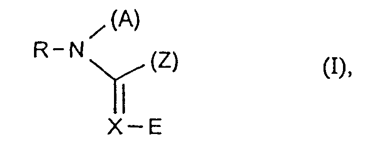 dans laquelle
R désigne un groupe hétéroarylméthyle ayant jusqu'à 6 atomes dans le noyau et N, O, S comme hétéroatomes, portant éventuellement un substituant alkyle ayant 1 à 4 atomes de carbone, alkoxy ayant 1 à 4 atomes de carbone, alkylthio ayant 1 à 4 atomes de carbone, halogénalkyle ayant 1 à 4 atomes de carbone et 1 à 5 atomes d'halogènes identiques ou différents, hydroxy, halogéno, cyano, nitro, amino, mono-alkyl- et dialkylamino ayant 1 à 4 atomes de carbone par groupe alkyle, ou représente l'hydrogène,
A est l'hydrogène ou un groupe alkyle ayant 1 à 4 atomes de carbone,
E est un groupe NO₂ ou CN,
X est un groupe =CH- ou =N-,
Z est un groupe alkyle ou un groupe 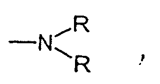 et
A et Z Z forment conjointement avec les atomes auxquels ils sont liés un noyau hétérocyclique pentagonal à heptagonal saturé ou non saturé, qui peut encore contenir 1 ou 2 hétéroatomes et/ou hétérogroupes identiques ou différents, en considérant comme hétéroatomes l'oxygène, le soufre ou l'azote et comme hétérogroupes des groupes N-alkyle, le radical alkyle d'un groupe N-alkyle contenant de préférence 1 à 4 atomes de carbone, à l'exception des composés (E)-N¹-((6-chloro-3-pyridyl)méthyl)-N²-cyano-N¹-méthylacétamidine (acétamipride), (E)-N-((6-chloro-3-pyridyl)méthyl)-N-éthyl-N'-méthyl-2-nitrovinylidène-diamine (nitenpyram) et 1-(6-chloro-3-pyridylméthyl)-N-nitro-imidazolidine-2-ylidène-amine (imidachlopride).

10. Agent suivant la revendication 1, contenant du fipronil et au moins l'un des composés du groupe de composés suivant :

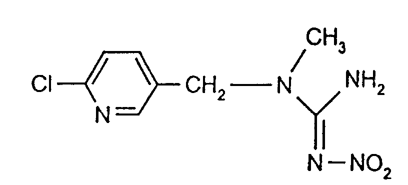

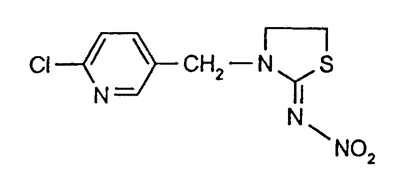

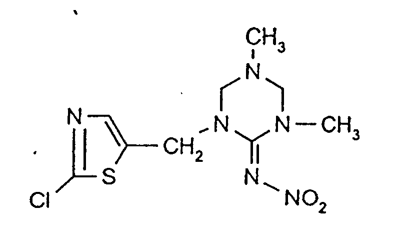

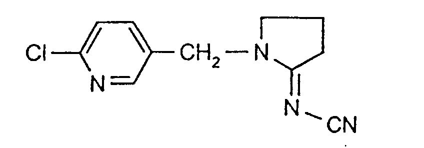

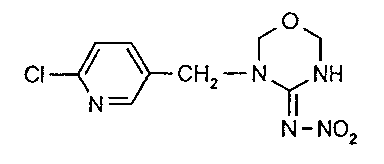

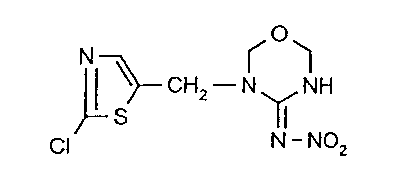

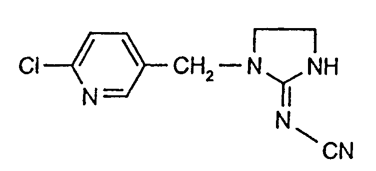

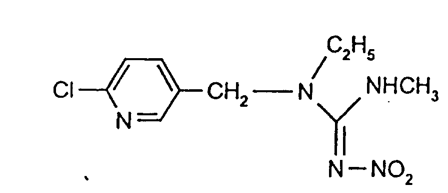

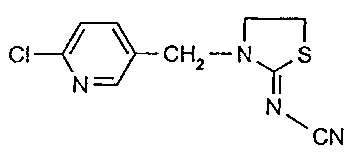

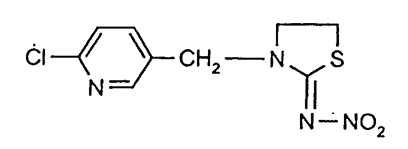

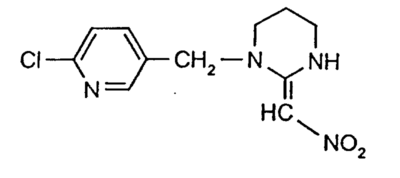

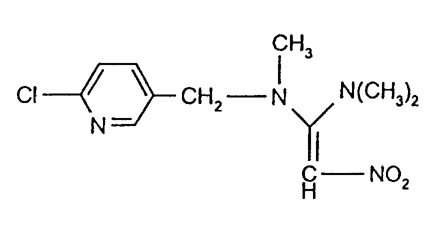

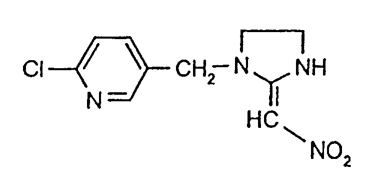

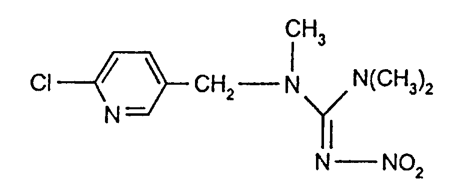

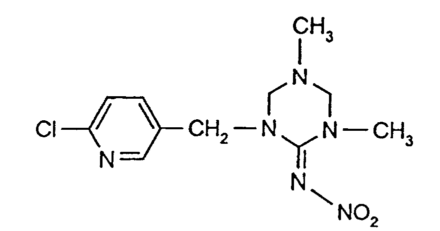

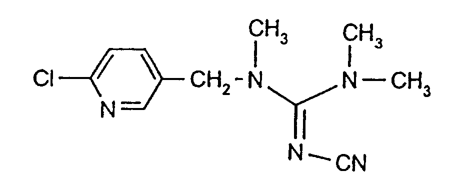

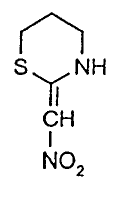

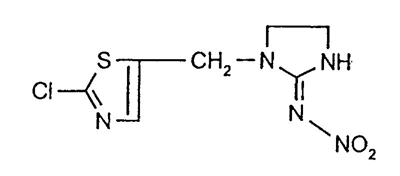

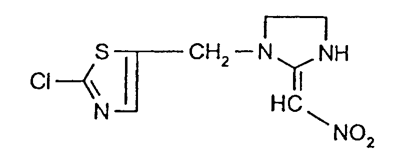

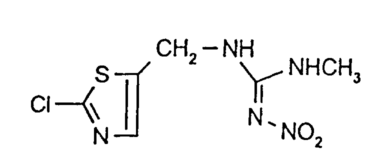

11. Agent suivant la revendication 1, contenant du fipronil et au moins un composé de formule (IIa), (IIb), (IIc), (IId), (IIf) ou (IIh)

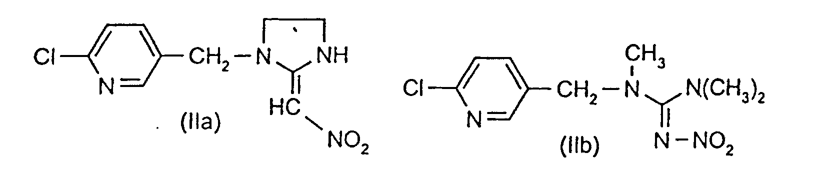

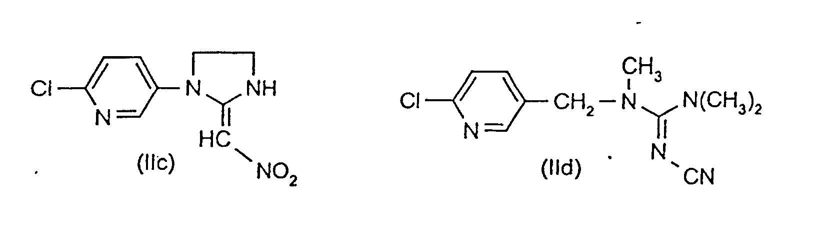

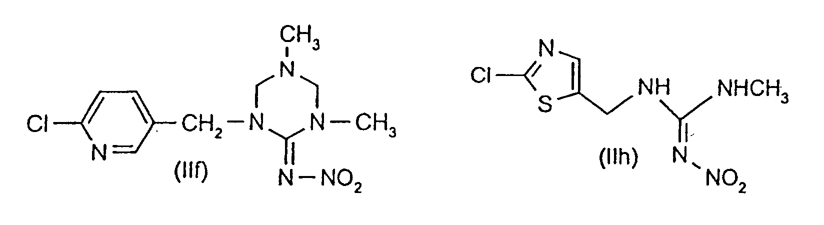

12. Agent suivant la revendication 9, contenant du fipronil et au moins l'un des composés de formule (I) dans un rapport de 1:100 à 100:1.

13. Procédé de préparation de compositions pour la protection du bois, **caractérisé en ce qu'**on mélange des agents suivant l'une des revendications 9 à 12 avec des composants classiques pour agents de protection du bois et, le cas échéant, d'autres substances actives.

14. Utilisation d'un agent contenant une quantité à effet synergique de filpronil et le composé de formule 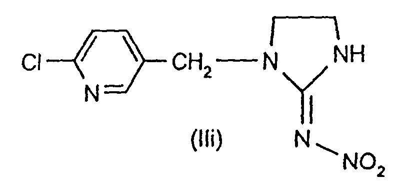 pour la protection de bois, de produits à base de bois et de matériaux à base de bois.
